# EUROPEAN PATENT APPLICATION

(11) **EP 1 988 178 A2**
(43) Date of publication of application: **05.11.2008**
(21) Application number: 08250943.1
(22) Date of filing: 18.03.2008
(51) Int. Cl.: C12Q 1/68

(54) **Nucleotide primer set and nucleotide probe for detecting genotype of serum amyloid A1(SAA1)**

(30) Priority: 26.04.2007 JP 2007117346
(71) Applicant: Kabushiki Kaisha Toshiba, Minato-ku, Tokyo 105-8001 (JP)
(72) Inventor: Nakamura, Naoko, c/o IP Division, Toshiba Corporation, Minato-ku, Tokyo 105-8001 (JP); Ito, Keiko, c/o IP Division, Toshiba Corporation, Minato-ku, Tokyo 105-8001 (JP); Takahashi, Masayoshi, c/o IP Division, Toshiba Corporation, Minato-ku, Tokyo 105-8001 (JP); Hashimoto, Koji, c/o IP Division, Toshiba Corporation, Minato-ku, Tokyo 105-8001 (JP); Gemma, Nobuhiro, c/o IP Division, Toshiba Corporation, Minato-ku, Tokyo 105-8001 (JP)
(74) Representative: Granleese, Rhian Jane

(57) **Abstract**

Provided is a LAMP-amplification nucleotide primer set for detection of the genotype of single nucleotide polymorphisms C-13T, C2995T and T3010C of the SAA1 gene. Also provided is a nucleotide probe for detection of the amplification product amplified with the primer set according to the present invention. Further provided is a method of detecting the genotype of the single nucleotide polymorphisms C-13T, C2995T and T3010C of the SAA1 gene by using the primer set according to the present invention.

## Description

The present invention relates to a nucleotide primer set and a detection probe for detecting a genotype of a single-nucleotide polymorphism in the serum amyloid A1 (SAA1) gene.

Amyloidosis is a disease involving accumulation of a filamentous abnormal protein called amyloid in the body causing malfunction of individual organs. It is a high-mortality disease for which there is no established fundamental therapy. Recent studies showed that it was possible to predict the propensity of the development of amyloidosis by examining the polymorphism of C-13T in the upstream region and C2995T and T3010C in the Exon3 region of the serum amyloid A1 (SAA1) gene. It is also possible to perform pharmaceutical administration and treatment tailor-made to the respective patient, by determining the genotype of the SAA1 gene.

The single-nucleotide polymorphism is generally detected by amplifying a target nucleotide with the Polymerase chain reaction (PCR) method and detecting wild-type and variant amplification products with a specific probe (see, the reference "Jain K. K., Application of Amplicip. CYP450, Mol Diagn. 9, 119-27 (2005)"). However, the PCR method has disadvantages such as complicated procedure of pretreatment including nucleotide extraction, demand for a complex temperature-regulating device such as a thermal cycler, and a longer reaction period of two hours or more. Amplification products by the PCR method are double-stranded chains, and thus, there is a problem in that the complementary chain degrades the detection sensitivity, while functioning as a competitor to the probe during detection. Various methods of converting the amplification product into a single-stranded chain, for example, by decomposing or separating a complementary chain by using an enzyme or magnetic beads, have been studied, but these methods also have a problem in that the operation is complicated and expensive.

According to one aspect of the present invention, there is provided a nucleotide primer set for LAMP amplification, used for detecting a genotype of a single-nucleotide polymorphism C-13T of a SAA1 gene, wherein when a target nucleotide has F3 region, F2 region and F1 region in turn from a 5' terminal and B3c region, B2c region and B1c region in turn from a 3' terminal, and when there are nucleotide primers including an FIP primer having a sequence identical with that of the F2 region in the 3' terminal side and a sequence complementary to the F1 region in the 5' terminal side, an F3 primer having a sequence identical with that of the F3 region, a BIP primer having a sequence complementary to the B2c region in the 3' terminal side and a sequence identical with that of the B1c region in the 5' terminal side, and a B3 primer having a sequence complementary to the B3c region, wherein the primer set comprising: an FIP primer and a BIP primer selected from the primer sets 1 to 7 shown in Table 2; an F3 primer binding to a region within 60 bases from the 5' terminal of the F2 region of the target nucleotide; and a B3 primer binding to a region within 60 bases from the 3' terminal of the B2c region of the target nucleic acid.

The primer sets 1 to 7 shown in Table 2 are as follows:
Primer set 1: FIP primer of SEQ ID No. 1 and BIP primer of SEQ ID No. 2,
Primer set 2: FIP primer of SEQ ID No. 1 and BIP primer of SEQ ID No. 3,
Primer set 3: FIP primer of SEQ ID No. 1 and BIP primer of SEQ ID No. 5,
Primer set 4: FIP primer of SEQ ID No. 1 and BIP primer of SEQ ID No. 6,
Primer set 5: FIP primer of SEQ ID No. 1 and BIP primer of SEQ ID No. 7,
Primer set 6: FIP primer of SEQ ID No. 1 and BIP primer of SEQ ID No. 10, and
Primer set 7: FIP primer of SEQ ID No. 13 and BIP primer of SEQ ID No. 3.

According to another aspect of the present invention, there is provided a nucleotide primer set for LAMP amplification, used for detecting a genotype of a single-nucleotide polymorphism C2995T and/or T3010C of the SAA1 gene, wherein the primer set comprising: an FIP primer and a BIP primer selected from the primer sets 1 to 4 shown in Table 3; an F3 primer binding to a region within 60 bases from the 5' terminal of the F2 region of the target nucleotide; and a B3 primer binding to a region within 60 bases from the 3' terminal of the B2c region of the target nucleic acid.

The primer sets 1 to 4 shown in Table 3 are as follows:
Primer set 1: FIP primer of SEQ ID No. 19 and BIP primer of SEQ ID No. 20,
Primer set 2: FIP primer of SEQ ID No. 21 and BIP primer of SEQ ID No. 20,
Primer set 3: FIP primer of SEQ ID No. 22 and BIP primer of SEQ ID No. 20, and
Primer set 4: FIP primer of SEQ ID No. 23 and BIP primer of SEQ ID No. 20.

According to another aspect of the present invention, there is provided a method of detecting a genotype of a single-nucleotide polymorphism C-13T, C2995T or T3010C of the SAA1 gene, comprising the steps of: obtaining an amplification product by amplifying a target nucleotide by using the nucleotide primer set; and measuring and comparing amounts of a wild-type amplification product and a variant amplification product contained in the amplification product.

Another aspect of the invention provides nucleotide probe used for detecting the amplified product obtained by amplification of a target nucleotide by using the nucleotide primer set comprising: a nucleotide probe selected from a wild-type nucleotide probe and a variant nucleotide probe.

In an aspect, the wild-type nucleotide probe is complementary to the wild-type amplification product and has a Tm value of 63 to 77°C; the variant nucleotide probe is complementary to the variant amplification products and has a Tm value of 63 to 74°C; and the single nucleotide polymorphism C-13T site is located inside by three or more bases from the terminal of respective nucleotide probes.

In another aspect, the wild-type nucleotide probe has a Tm value of 63 to 74°C; the variant nucleotide probe has a Tm value of 61 to 74°C; and the single nucleotide polymorphism C2995T site is located inside by three or more bases from the terminal of respective nucleotide probes.

In a further aspect, the wild-type nucleotide probe has a Tm value of 55 to 70°C; the variant nucleotide probe has a Tm value of 55 to 71°C; and the single nucleotide polymorphism T3010C site is located inside by three or more bases from the terminal of respective nucleotide probes.

Another aspect of the invention provides a method for detecting the genotype of the single-nucleotide polymorphisms C-13T, C2995T and T3010C of the SAA1 gene simultaneously.

The present invention provides a nucleotide primer and detection probe for detecting the genotype of the single-nucleotide polymorphism of the SAA1 gene. It is thus possible to detect the genotype of the single-nucleotide polymorphisms sites of C-13T, C2995T and T3010C of the SAA1 gene easily and cost-effectively.

The invention can be more fully understood from the following detailed description when taken in conjunction with the accompanying drawings, in which:
FIG. 1 is a schematic diagram showing the LAMP method;
FIG. 2 is a schematic chart showing an intermediate product and the annealing site of an inner primer (FIP or BIP) by the LAMP method;
FIG. 3 is a schematic diagram showing the location of loop primers;
FIG. 4 is a schematic chart showing an intermediate product and the annealing site of a loop primer (LFc or LBc) by the LAMP method;
FIG. 5 is a schematic diagram showing the detection position of the amplified product;
FIG. 6 is a schematic planar view of an embodiment of a probe-immobilized support;
FIG. 7 is a schematic planar view of an embodiment of the probe-immobilized support;
FIG. 8 is a schematic diagram showing the location of the BIP primer;
FIG. 9 is an electropherogram of the amplification products obtained by using the primer set;
FIG. 10 shows an electrophoretogram of the amplification products including non-specific amplification product;
FIG. 11 shows graphs indicating the test results 1 obtained by using the probe for detection of C-13T;
FIG. 12 shows graphs indicating the test results 2 obtained by using the probe for detection of C-13T;
FIG. 13A shows graphs indicating the test results 3 (wild-type) obtained by using the probe for detection of C-13T;
FIG. 13B shows graphs indicating the test results 3 (variant) obtained by using the probe for detection of C-13T;
FIG. 13C shows graphs indicating the test results 3 (heterozygous) obtained by using the probe for detection of C-13T;
FIG. 14 shows graphs indicating the test results 1 obtained by using the probe for detection C2995T;
FIG. 15 shows graphs indicating the test results 2 obtained by using the probe for detection C2995T;
FIG. 16 shows graphs indicating the test results obtained by using the probe for detection T3010C; and
FIG. 17 shows graphs indicating the results of simultaneous detection of C-13T, C2995T, and T3010C.

The PCR method has been frequently used for detection of a single-nucleotide polymorphism, but the PCR method has the disadvantages described above. Thus in the present invention, the single-nucleotide polymorphism is detected by the loop-mediated isothermal amplification (LAMP) method, replacing the PCR method. In the LAMP method, a nucleotide is amplified under an isothermal condition at 60 to 65°C. The LAMP method has the advantage that it is possible to obtain amplification products in a shorter period in a greater amount than the PCR method. It is also reported that the reaction is less influenced by impurities in a sample. It is therefore possible to amplify a target nucleotide easily by the LAMP method.

In an embodiment of the present invention, a target nucleotide is amplified by the LAMP method, and the amounts of the wild-type amplification products and variant amplification products in the amplification products obtained are respectively determined. When there are many wild-type amplification products, the genotype of the tested target nucleotide can be judged as a wild-type homo. On the contrary, when there are many variant amplification products, the target nucleotide can be judged as a variant homo. Alternatively, when there are almost the same amounts of the wild-type and variant amplification products, the target nucleotide can be judged as a heterozygous.

The amounts of the amplification products are determined, for example, by using a nucleotide probe. The nucleotide probes include one complementary to wild-type amplification products and one complementary to variant amplification products. Amplification products and the respective nucleotide probes are allowed to hybridize to each other, and the amounts of the amplification products bound to respective nucleotide probes are determined. It is possible to determine the genotype of the target nucleotide by comparing the amounts of the amplification products bound to a wild-type nucleotide probe and the amounts of the amplification products bound to a variant nucleotide probe.

### <Summary of LAMP method>

Hereinafter, the LAMP method will be described briefly. In the present specification, the nucleotide (including genomic DNA or the like) subjected to detection of a single-nucleotide polymorphism will be called analyte nucleotide. The region in the SAA1 gene amplified by the LAMP method will be called a target nucleotide. The products obtained by the LAMP method will be called amplification products. The solution containing human genomic DNA will be called a sample solution.

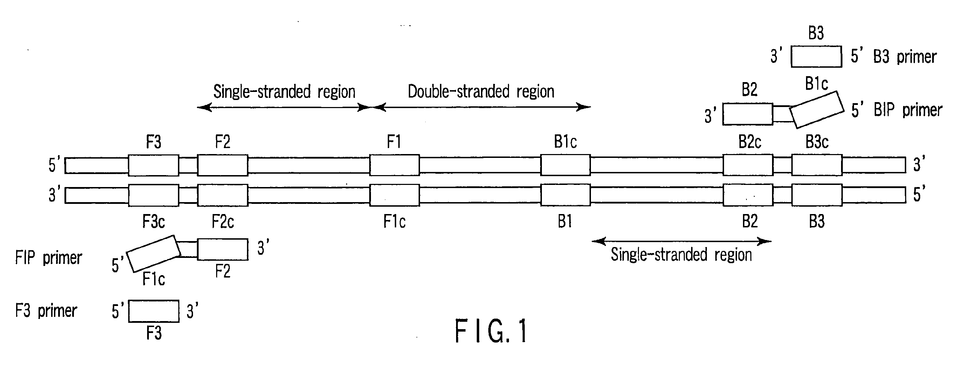

In the LAMP method, the target nucleotide is designed to have F3 region, F2 region, and F1 region in turn from the 5' terminal and B3c region, B2c region, and B1c region in turn from the 3' terminal. The target nucleotides are amplified by using the four kinds of primers shown in FIG. 1. The regions F1c, F2c, F3c, B1, B2, and B3 are regions of the complementary chains respectively corresponding to the regions F1, F2, F3, B1c, B2c, and B3c.

The four kinds of primer used for amplification of the nucleotide in the LAMP method are (1) FIP primer having the sequence identical with the F2 region in the 3' terminal side and the sequence complementary to the F1 region in the 5' terminal side; (2) F3 primer having the sequence identical with the F3 region; (3) BIP primer having the sequence complementary to the B2c region in the 3' terminal side and the sequence identical with the B1c region in the 5' terminal side; and (4) B3 primer having the sequence complementary to the B3c region. Generally, FIP and BIP primers are called inner primers, while F3 and B3 primers are called outer primers.

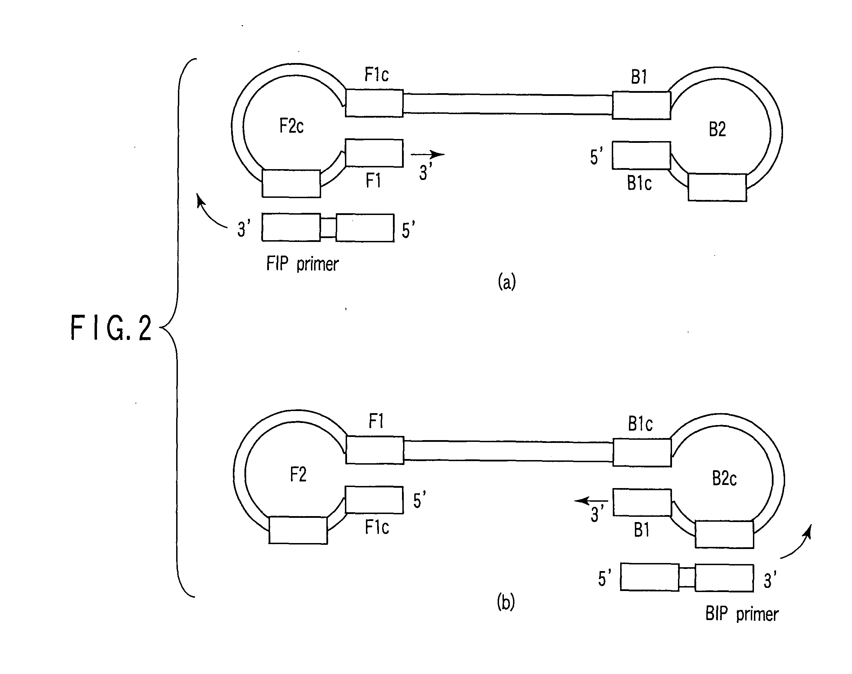

LAMP amplification by using the four kinds of primers gives intermediate products having the dumbbell structure shown in FIG. 2. FIP and BIP primers bind to F2c region and B2c region in single-stranded loops, and extending reaction proceeds from the 3' terminal of the each primer and the 3' terminal of the intermediate product itself. See Japanese Patent No. 3313358 for details.

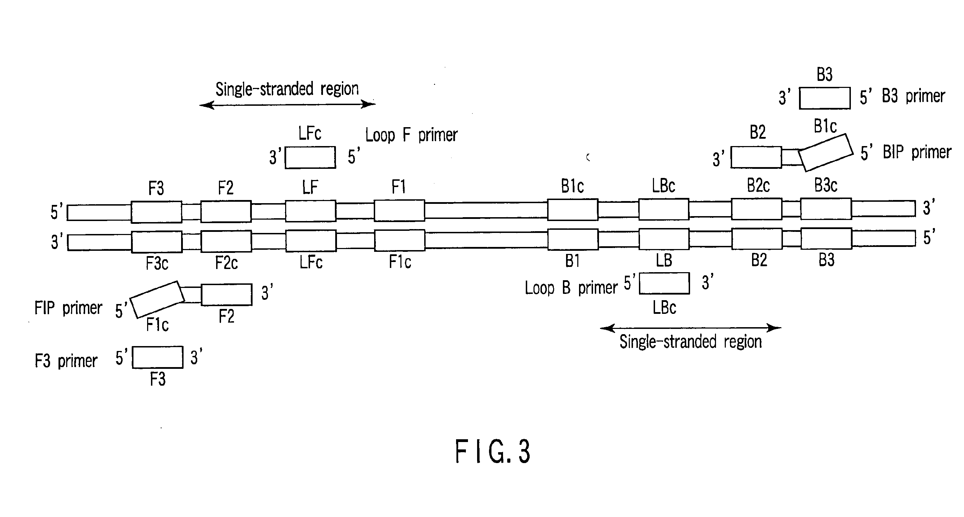

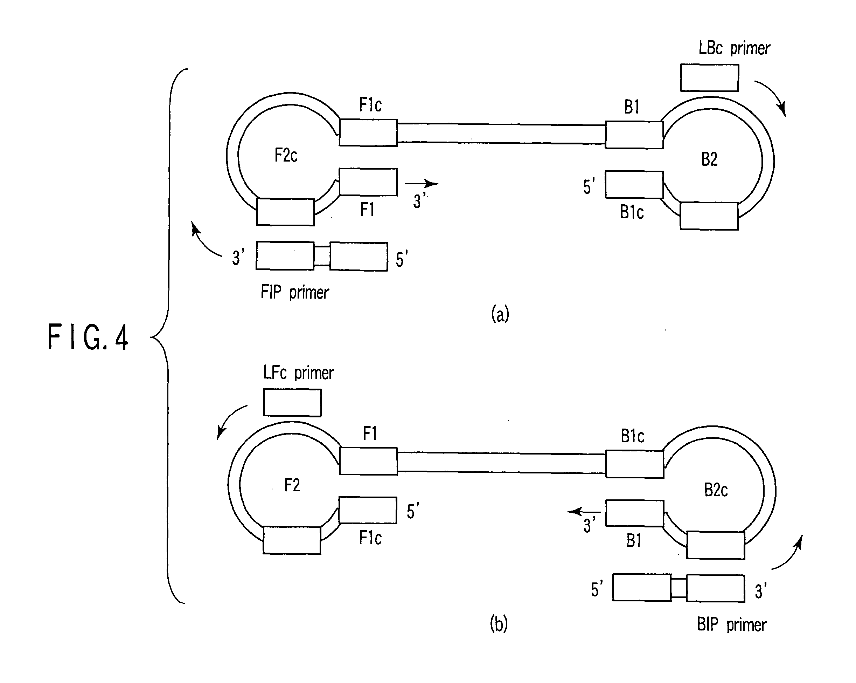

It is possible to shorten the amplification period by using a primer, called a loop primer, optionally in the LAMP method. In such a case, as shown in FIG. 3, a LF region is designed in the region from F2 region to F1 region and a LBc region is designed in the region from B2c region to B1c region. These regions are called loop primer region. In addition to the four kinds of primers, a loop primer LFc having the sequence complementary to the LF region and a loop primer LBc having the sequence identical with the LBc region are used. See WO2002/024902 for details. These loop primers LFc and LBc may be used simultaneously, or alternatively, only one of them may be used. The loop primers are annealed to a loop different from the loop to which FIT and BIP primers are annealed, as shown in FIG. 4, giving additional synthetic origins and thus accelerating amplification.

### <Detection of LAMP amplification products; nucleotide probe>

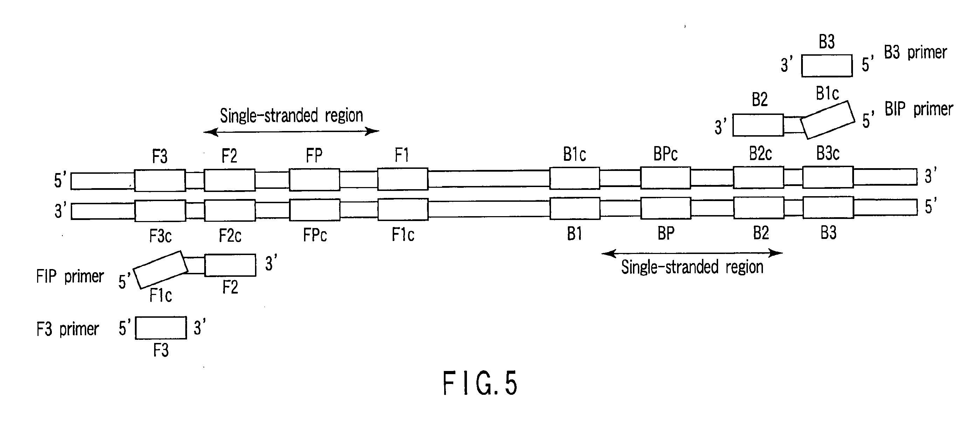

For detection of a single-nucleotide polymorphism, the polymorphic site to be detected is located in the FP region or BPc region shown in FIG. 5. Alternatively, different polymorphisms may be located in the FP region and BPc region, respectively. As shown in FIG. 5, the region from F2 region to F1 region is a part that becomes single stranded in the amplification product. Similarly, the region from B2c region to B1c region is also a part that becomes single-stranded in the amplification product. It is possible to make the detection by nucleotide probe easier, by locating the polymorphic site to be detected in a single-stranded part.

The nucleotide probe is designed to bind to the FP region or BPc region containing the polymorphic site. Thus, the nucleotide probe has a sequence complementary to that of the region containing the polymorphic site among FP region or BPc region.

FPc region complementary to FP region and BP region complementary to BPc region is also present in the amplification products. Accordingly, it is possible to use the FPc region and BP region for detection.

In the present specification, nucleotide probes containing sequences complementary to that of wild-type amplification products are called wild-type nucleotide probes, while the nucleotide probes containing the sequences complementary to that of variant amplification products are called variant nucleotide probes.

The nucleotide probe may be consisted of, but is not limited to, DNA, RNA, PNA, LNA, a nucleotide having a methyl phosphonate skeleton, or other synthetic nucleotide chain. For immobilization on a support, the terminal thereof may be modified with a reactive functional group such as amino, carboxyl, hydroxyl, thiol, or sulfonyl group. A spacer may be introduced between the functional group and the nucleotide. The spacer may have, for example, an alkane skeleton, an ethylene glycol skeleton, or the like.

### <Nucleotide probe-immobilized support>

The nucleotide probe may be used, for example, as it is immobilized on a support. The nucleotide probe-immobilized support may be used in a known apparatus such as a so-called DNA chip and DNA microarray.

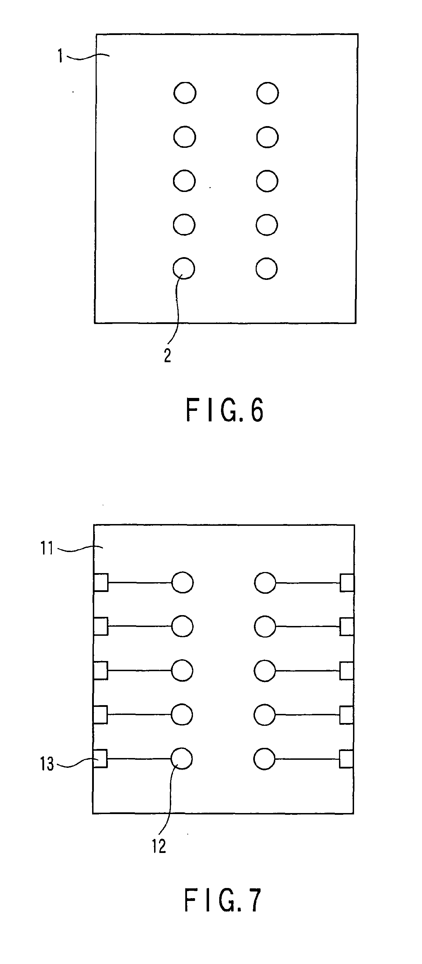

An embodiment of the probe-immobilized support is shown in FIG. 6. The probe is immobilized in an immobilization area 2 of a support 1. The support 1 may be prepared with a silicon substrate, but is not limited thereto. The probe may be immobilized by any known means. Only one kind of probe may be immobilized on a support, or different kinds of probe may be immobilized on a support, the location and the number thereof may be modified as needed by those who are skilled in the art. As will be described below, a probe-immobilized support according to the present embodiment may be used for fluorescent detection of a probe.

A schematic view of the probe-immobilized support in another embodiment is shown in FIG. 7. In the present embodiment, electrodes 12 are formed on a support 11. The probe is immobilized on the electrode 12. Each electrode 12 is connected to a pad 13 for extracting electrical information. The support 11 may be prepared, for example, from a silicon substrate, but is not limited thereto. Production of electrode and immobilization of probe may be performed by any known means. The electrode can be made of any material which include, but not limited to, single metals such as gold, gold alloy, silver, platinum, mercury, nickel, palladium, silicon, germanium, gallium and tungsten, alloys thereof, carbons such as graphite and glassy carbon, and the oxides or compounds thereof.

The immobilization support shown in FIG. 7 has 10 electrodes, but the number of the electrodes formed on a single support is not limited thereto and may be modified optionally. The positional pattern of the electrodes is also not limited to that shown in the figure, and may be modified as needed by those who are skilled in the art. Reference electrodes and counter electrodes may be formed as needed on the support 1. As will be described below, a probe-immobilized support according to this embodiment may be used for electrochemical detection.

### <Hybridization of nucleotide probe with amplification product>

The amplification products are hybridized to the nucleotide probe under a suitable condition. The suitable condition varies according to the kind and structure of the amplification products, the kind of nucleotide contained in the sequence to be detected, and the kind of nucleotide probe. Specifically, the hybridization is performed in a buffer solution at an ionic strength in the range of 0.01 to 5 and at a pH in the range of 5 to 10. Dextran sulfate, which is a hybridization accelerator, salmon sperm DNA, bovine thymic DNA, EDTA, a surfactant, and the like may be added to the reaction solution. The reaction temperature is, for example, in the range of 10 to 90°C, and the reaction mixture may be stirred or shaken to improve the reaction efficiency. After reaction, a buffer solution for example at an ionic strength in the range of 0.01 to 5 and at a pH in the range of 5 to 10 may be used for washing.

### <Detection method>

Hybridization between the probe immobilized on the support and the amplification products gives double-strand nucleotides. The double-strand nucleotides can be detected electrochemically or by fluorescence.

### (a) Current detection system

Electrochemical detection of the double-strand nucleotide will be described below. The method uses a double-stranded chain-recognizing compound that recognizes a double-strand nucleotide specifically. Examples of the double-stranded chain-recognizing compounds include, but are not limited to, Hoechst 33258, acridine orange, quinacrine, daunomycin, metallointercalators, bisintercalators such as bisacridine, trisintercalators, and polyintercalators. These substances may be modified with an electrochemically active metal complex such as ferrocene or viologen.

The concentration of the double-stranded chain-recognizing compound may vary according to its kind, but generally, it is used at a concentration range of 1 ng/mL to 1 mg/mL. In this case, a buffer solution at an ionic strength in the range of 0.001 to 5 and at a pH in the range of 5 to 10 is used preferably.

A double-stranded chain-recognizing compound is added to the reaction solution during or after a hybridization reaction. The double-stranded chain-recognizing compound binds to double-strand nucleotides, if formed by hybridization. For example, it is possible to measure the reaction current derived from the double-stranded chain-recognizing compound, by applying an electric potential higher than that causing electrochemical reaction of the double-stranded chain-recognizing compound. In this case, the electric potential may be applied at a constant velocity, or applied in a pulse shape or at a constant voltage. The current and voltage may be controlled during measurement by using a device such as a potentiostat, digital multimeter, or function generator. For example, the known electrochemical detecting means disclosed in JP-A 10-146183 (KOKAI) is used preferably.

### (b) Fluorescent detection method

The method of detecting a double-strand nucleotide by fluorescence will be described below. The primer is previously labelled with a fluorescently active substance. Alternatively, it is detected with a secondary probe labelled with a fluorescently active substance. A secondary probe with multiple labels may be used. Examples of the fluorescently active substance include, but are not limited to, fluorescent colorants such as FITC, Cy3, Cy5, and rhodamine. The fluorescent material is detected, for example, with a fluorescence detector. A detector suitable for the kind of label is used for detection of labeled sequence to be detected or secondary probe.

### <Selection of nucleotide primer>

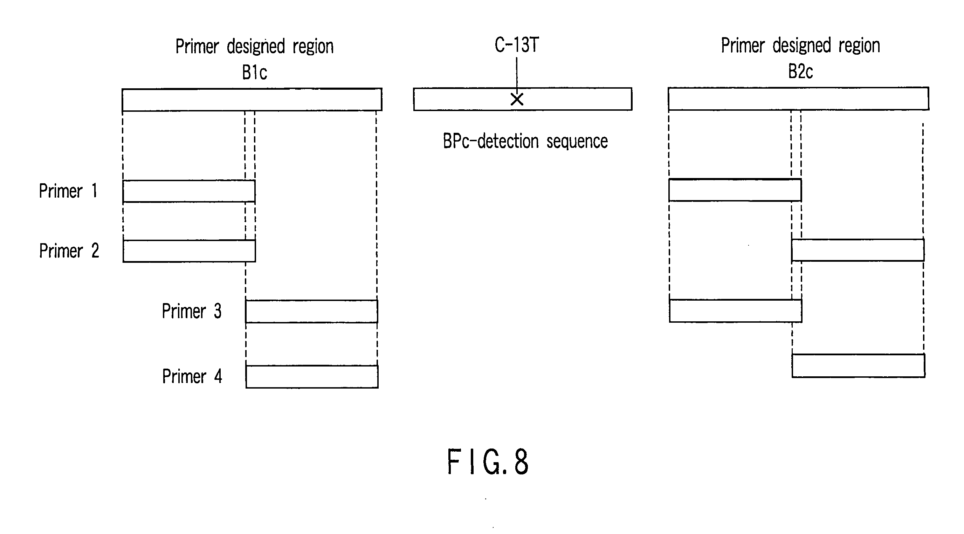

FIG. 8 is a schematic diagram showing nucleotide primers when a single-nucleotide polymorphism C-13T is located in the region between B1c and B2c, i.e., BPc region. The FIP primer has the same sequence as the B1c region and the sequence complementary to that of the F2 region. Various kinds of primers may be designed, as long as the B2c region and B1c region is positioned at places holding C-13T inside.

However, studies by the inventors have revealed that the amplification efficiency by the LAMP method varies according to the kind of primer. For example, amplification is performed by using primer one of the four kinds of primers shown in FIG. 8, and common three primers (BIP, F3, and B3 primers). As a result, the sample with primer 1 is not amplified even after a sufficient period, for example after 2 hours. The sample with primer 2 is amplified after approximately 1 hour, but non-specific amplification of primers is occurred. The sample with primer 3 does not cause non-specific amplification of primers, but requires an amplification period of 1.5 to 2 hours. The sample with primer 4 causes no non-specific amplification of primers and completes amplification within 1 hour. In this case, the primers preferable for amplification are the primers 3 and 4, and the best primer is the primer 4. Thus, it is superior primer that is higher in amplification efficiency, allows amplification in a short period of time, and causes no non-specific amplification.

For detection of amplification products with a nucleotide probe, the amplification product preferably hybridizes with the nucleotide probe at a high efficiency. Accordingly, the hybridization efficiency of the amplification products is also considered in evaluating the primer.

In addition, the human SAA1 gene is highly homologous to human SAA2, SAA3, and SAA4 genes. Accordingly for specific amplification of SAA1, any one or more of the regions for the inner primer, i.e., F2, F1, B1c and B2c regions, should be designed to be located in a region where the SAA1 gene sequence is different from the three gene sequences above. In addition, the inner, outer, and loop primers are preferably not placed at the site where mutation is reported. If a primer is to be optionally designed in the mutation position, it is preferable to introduce a mix base or a universal base such as deoxyinosine (dI).

Another inner primer having no single-nucleotide polymorphism inside is preferably placed in a region having a F2 to B2 length of 450 bp or less, more preferably 350 bp or less. In addition, both inner primers are preferably designed to make the length of the single-stranded loop 100 bp or less, more preferably 70 bp or less.

Non-specific amplification among primers is a phenomenon often found in the LAMP reaction. The FIP primer, which contains the F1c region and F2 region, is often a long-chain nucleotide. Similarly, the BIP primer, which contains the B1c region and B2 region, is often a long-chain nucleotide. Thus, among FIP primers, among BIP primers, or FIP and BIP primers may be entangled with each other, frequently allowing amplification of which template is primer. The possibility of a non-specific reaction is higher in the LAMP reaction than in the PCR reaction, because the reaction solution contains the F3 and B3 primers and additionally LFc and LBc primers in some cases. Such non-specific reaction leads to a decrease in the amount of desirable LAMP products obtained by using the analyte nucleotide as the template.

If a non-specific reaction occurs in a negative control reaction solution containing no added analyte nucleotide, it is not possible to determine whether the white precipitate of pyrophosphoric acid and Mg released along with progress of amplification is caused by non-specific amplification or by contamination. Accordingly, it is important to eliminate the primers possibly causing non-specific amplification.

Accordingly, the inventors have conducted tests for selection of the nucleotide primer most preferable for amplification of a single-nucleotide polymorphism C-13T of the SAA1 gene, and also for amplification of a single-nucleotide polymorphism C2995T and T3010C.

### [Test 1: Primer for C-13T]

An amplification reaction was performed at 63°C for 1 hour or 2 hours, by using 12 kinds of nucleotide primer sets containing the SAA1 C-13T polymorphic site in the FP region. The composition of the reaction solution is shown in Table 1. The template DNA used was a human genome. For examination of the presence or absence of contamination and non-specific amplification, a negative control containing sterilized ultrapure water instead of the human genome was prepared in all sets. After the amplification reaction, amplification products were identified by 3% agarose electrophoresis.

The nucleotide primer sets used are shown in Table 2.

**Table 1 LAMP reaction composition**

| Bst DNA Polymerase | | 1 *µ*L |
|---|---|---|
| 2 × Buffer | | 12.5 *µ*L |
| | Tris·HCl pH8.0 40 mM | |
| | KCl 20 mM | |
| | MgSO₄ 16 mM | |
| | (NH₄)₂SO₄ 20 mM | |
| | Tween20 0.2% | |
| | Betaine 1.6M | |
| | dNTP 2.8 mM | |
| F3 primer (10 µM) | | 0.5 µL |
| B3 primer (10 µM) | | 0.5 µL |
| FIP primer (40 µM) | | 1 µL |
| BIP primer (40 µM) | | 1 µL |
| LFc primer (20 µM) | | 1 µL |
| Human genome (30 ng/µL*)* | | 1 µL |
| Sterilized ultrapure water | | 6.5 µL |
| Total | | 25 µL |

**Table 2 Twelve nucleotide primer sets designed to have C-13T in the BP or BPc region**

| Primer set | SEQ ID No. | Name | Sequence | 1-hour amplification | 2-hours amplification | Non-specific amplification |
|---|---|---|---|---|---|---|
| 1 | 1 | FIP-1 | CAGTGGTTTCTTCATCCCGCAGGCACATCTTGTTCCCTC | ○ | ○ | Not-occurred |
| | 2 | BIP-1 | GGAAGGCTCAGTATAAATAGCA TAGCTGAGCTGCGGGTCCCT | | | |
| 2 | 1 | FIP-1 | CAGTGGTTTCTTCATCCCGCAGGCACATCTTGTTCCCTC | ○ | ○ | Not-occurred |
| | 3 | BIP-2 | GGAAGGCTCAGTATAAATAGCAGTGCTGTAGCTGAGCTGCGG | | | |
| 8 | 1 | FIP-1 | CAGTGGTTTCTTCATCCCGCAGGCACATCTTGTTCCCTC | × | ○ | Occurred |
| | 4 | BIP-3 | GGAAGGCTCAGTATAAATAGCAACCTGATCTGTGCTGTAGCTG | | | |
| 3 | 1 | FIP-1 | CAGTGGTTTCTTCATCCCGCAGGCACATCTTGTTCCCTC | ○ | ○ | Not-occurred |
| | 5 | BIP-4 | GGAAGGCTCAGTATAAATTGTAGCTGAGCTGCGGGTCC | | | |
| 4 | 1 | FIP-1 | CAGTGGTTTCTTCATCCCGCAGGCACATCTTGTTCCCTC | ○ | ○ | Not-occurred |
| | 6 | BIP-5 | GGAAGGCTCAGTATAAATGTGCTGTAGCTGAGCTGCGG | | | |
| 5 | 1 | FIP-1 | CAGTGGTTTCTTCATCCCGCAGGCACATCTTGTTCCCTC | × | ○ | Not-occurred |
| | 7 | BIP-6 | GGAAGGCTCAGTATAAATACCTGATCTGTGCTGTAGCTG | | | |
| 9 | 1 | FIP-1 | CAGTGGTTTCTTCATCCCGCAGGCACATCTTGTTCCCTC | × | × | Not-occurred |
| | 8 | BIP-7 | GGAAGGCTCAGTATAAATAGCACTCCTCACCTGATCTGTGC | | | |
| 10 | 1 | FIP-1 | CAGTGGTTTCTTCATCCCGCAGGCACATCTTGTTCCCTC | × | × | Not-occurred |
| | 9 | BIP-8 | GGAAGGCTCAGTATAAATAGCACTTGGTGTGCTCCTCACC | | | |
| 6 | 1 | FIP-1 | CAGTGGTTTCTTCATCCCGCAGGCACATCTTGTTCCCTC | ○ | ○ | Not-occurred |
| | 10 | BIP-9 | GGAAGGCTCAGTATAAATAGCAAAAATCACTCCTTGGTGTGC | | | |
| 11 | 11 | FIP-2 | CTGTCAGGGCAGGAGACCTCCGCAGCCTCAGC | × | × | Not-occurred |
| | 3 | BIP-2 | GGAAGGCTCAGTATAAATAGCAGTGCTGTAGCTGAGCTGCGG | | | |
| 12 | 12 | FIP-3 | GCTGTCAGGGCAGGAGACCCCGCAGCCTCAGCC | × | × | Not-occurred |
| | 3 | BIP-2 | GGAAGGCTCAGTATAAATAGCAGTGCTGTAGCTGAGCTGCGG | | | |
| 7 | 13 | FIP-4 | CTGCAGGTCATTTCCCCTACATCCAGGAACTTGTCTTAGACCGT | × | ○ | Not-occurred |
| | 3 | BIP-2 | GGAAGGCTCAGTATAAATAGCAGTGCTGTAGCTGAGCTGCGG | | | |
| | 14 | F3-1 | GTCTCCTGCCCTGACAGC | | | |
| | 15 | F3-2 | TCAGGAGCTGGCTTCAAAG | | | |
| | 16 | F3-3 | CAGGCACATCTTGTTCCCTC | | | |
| | 17 | B3-1 | ACTCCTTGGTGTGCTCCTC | | | |
| | 18 | B3-2 | AATGATAATCTTGTTGGGAAAGAG | | | |
| | 58 | LFc | GTCATTTATCCCAGTTGTGCAACC | | | |

F3 primer has the sequence of SEQ ID No. 14 for primer sets 1 to 6 and 8 to 10, has the sequence of SEQ ID No. 15 for primer sets 11 and 12, and has the sequence of SEQ ID No. 16 for primer sets 7. B3 primer has the sequence of SEQ ID No. 17 for primer sets 1 to 5, 7, 8, 11 and 12, and has the sequence of SEQ ID No. 18 for primer sets 6, 9 and 10.

### [Test 1: Results]

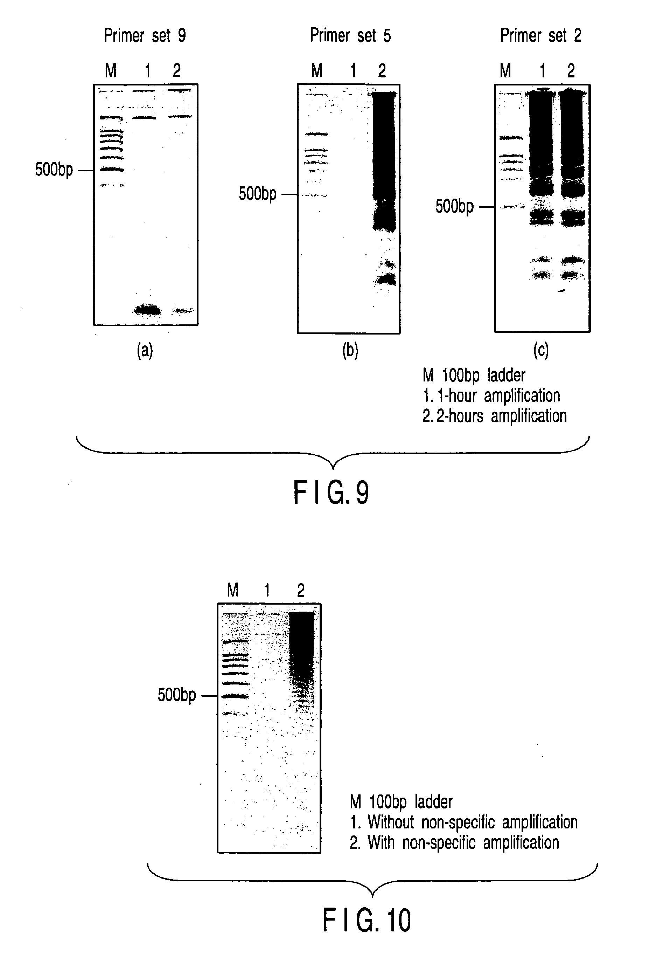

The amplification products obtained by using the 12 kinds of primer sets shown in Table 2 were subjected to electrophoresis. The results are summarized in FIG. 9. With the primer set 9, there was no amplification product obtained after amplification for 2 hour. With the primer set 5, there was no amplification product after reaction for 1 hour, but sufficient amount of amplification product was obtained after reaction for 2 hours. With the primer set 2, sufficient amount of amplification product was obtained after amplification for 1 hour. Similar tests were conducted with other primer sets. As a result, after amplification for 1 hour, sufficient amounts of amplification products were obtained with the primer sets 1, 2, 3, 4, and 6. After amplification for 2 hours, sufficient amounts of amplification products were obtained with the primer sets 1, 2, 3, 4, 5, 6, 7, and 8. With the primer sets 9, 10, 11, and 12, there were few amplification products, or no amplification was confirmed. Non-specific amplification was occurred with the primer set 8 as shown in FIG. 10. The results above showed that the primer sets 1, 2, 3, 4, 5, 6 and 7 were preferable, and that the primer sets 1, 2, 3, 4 and 6 were the most preferable. The results are summarized in Table 2.

### [Test 2: Primer for C2995T and T3010C]

In detection of neighboring single-nucleotide polymorphisms such as C2995T and T3010, it is possible to place two single-nucleotide polymorphisms in the same single-stranded loop region. Thus, it is possible to detect two single-nucleotide polymorphisms with a single amplification product, simplifying and reducing the cost of the detection method, compared to the case where two amplification products are prepared separately.

An amplification reaction was performed at 63°C for 1 hour or 2 hours, by using 4 kinds of nucleotide primer sets containing the SAA1 C2995T and T3010C polymorphic sites in the FP and FPc regions. The composition of the reaction solution is shown in Table 1. The template DNA used was a human genome. For examination of the presence or absence of contamination and non-specific amplification, a negative control containing sterilized ultrapure water instead of the human genome was prepared in all sets. After the amplification reaction, amplification products were identified by 3% agarose electrophoresis.

The nucleotide primer sets used are shown in Table 3.

**Table 3 Four nucleotide primer sets designed to have C2995T and T3010C in the FP or FPc region**

| Primer set | SEQ ID No. | Name | Sequence | 1-hour amplification | 2-hours amplification | Non-specific amplification |
|---|---|---|---|---|---|---|
| 1 | 19 | FIP-1 | CGTCCCAGGAGCTCTGCTCGGGGGAACTATGA | ○ | ○ | Not-occurred |
| | 20 | BIP-1 | CTGTTCATCCAGCCTAGGGGGATGAAAACACTGGGCACC | | | |
| 2 | 21 | FIP-2 | CGTCCCAGGAGCTCCTGCTCGGGGGAACTATGA | ○ | ○ | Not-occurred |
| | 20 | BIP-1 | CTGTTCATCCAGCCTAGGGGGATGAAAACACTGGGCACC | | | |
| 3 | 22 | FIP-3 | CGTCCCAGGAGCTCAGGGGACCTGGGGG | ○ | ○ | Not-occurred |
| | 20 | BIP-1 | CTGTTCATCCAGCCTAGGGGGATGAAAACACTGGGCACC | | | |
| 4 | 23 | FIP-4 | CGTCCCAGGAGCTCCAGGGGACCTGGGGG | ○ | ○ | Not-occurred |
| | 20 | BIP-1 | CTGTTCATCCAGCCTAGGGGGATGAAAACACTGGGCACC | | | |
| | 24 | F3 | CCAATTACATCGGCTCAGAC | | | |
| | 25 | B3 | TGAGCAATAGCCCACCC | | | |
| | 59 | LBc | AGCCTGGCTGAATGGG | | | |

F3 primer has the sequence of SEQ ID No. 24, and B3 primer has the sequence of SEQ ID No. 25; and both primers were used commonly in all sets.

### [Test 2: Results]

The amplification product obtained by using the 4 kinds of primer sets shown in Table 3 were subjected to electrophoresis. After amplification for 1 hour, all primer sets gave sufficient amounts of amplification products. Similarly, after amplification for 2 hours, all primer sets gave sufficient amounts of amplification products. No non-specific amplification was observed in any of the sets. The results above showed that the primer sets 1, 2, 3, and 4 were preferable. The results are summarized in Table 3.

The preferable inner primer provided by the present invention was determined by the above tests. As apparent for those who are skilled in the art, the primer most important in the LAMP reaction is the inner primer. An inner primer is essential for amplification reaction, but outer and loop primers are not. The addition of outer and loop primers to the amplification reaction solution may lead to an increase of amplification efficiency, but it's positional change gives less influence on amplification efficiency than that of the inner primer. Thus, the outer primers (F3 and B3 primers) can be designed optionally. For example, it is preferably placed in the region within 60 bases from the 5' terminal of the F2 region and the region within 60 bases from the 3' terminal of the B2c region. Thus, the F3 region having the same sequence as the F3 primer is preferably placed in the region within 60 bases from the 5' terminal of the F2 region, and the B3c region having a sequence complementary to the B3 primer in the region within 60 bases from the 3' terminal of the B2c region. The loop primer is preferably designed to bind to a loop other than the loop to which the inner primer binds, and the loop primer is not placed in the inner primer region.

### <Selection of nucleotide probe>

The chain of the nucleotide probe is neither too long nor too short. Generally, an increase in chain length leads to an increase in the binding force, although there is some difference in the binding force according to the kind of the base. An excessively small chain length of the nucleotide probe leads to deterioration of the hybridization efficiency between the nucleotide probe and amplification products. On the other hand, an excessively large chain length of the nucleotide probe leads to a decrease in one-base difference between the wild-type nucleotide probe and the variant nucleotide probe. Accordingly, non-specific bonding between wild-type amplification products and variant nucleotide probes and also between variant amplification products and wild-type nucleotide probes increases. Thus, it is preferable to use a nucleotide probe having an appropriate chain length, for example of 10 to 35 bases, for detection of single-nucleotide polymorphism.

The binding force may be indicated by the dissociation temperature Tm of the double-strand nucleotide. The Tm value is calculated, for example, by nearest neighbor method, Wallance method, or GC % method. In the present invention, used is the nearest neighbor method (Breslauer et. al., Proc. Natl. Acad. Sct. USA, Vol.83, pp.3746-3750, June 1986; Freier et. al., Proc. Natl. Acad. Sct. USA, Vol.83, pp.9373-9377, December 1986; Schildkraut et. al., BIOPOLYMERS, Vol.3, pp.195-208, 1965). In the invention, it is calculated under the condition of an Na⁺ concentration of 50 mM and a nucleotide probe (oligonucleotide) concentration of 0.5 µM.

Hereinafter, a test for selecting a nucleotide probe suitably used in detecting amplification products obtained according to the present invention will be described.

### [Test 3-1: Probe for detection of C-13T]

LAMP amplification was performed at 63°C for 1 hour, by using a human genome determined to be heterozygous by Polymerase chain reaction-restriction fragment length polymorphism (PCR-RFLP) analysis as a template and also the primer set 2 for detection of C-13T. The primer set 2 for detection of C-13T is a set determined to be the best primer in the test 1 above. The amplification products obtained were detected on a current-detection DNA chip.

### Nucleotide probe:

The nucleotide sequences of the nucleotide probes tested are summarized in Table 4. The nucleotide probe used was a plus chain. The 3' terminal of the nucleotide probe was thiol-modified for immobilization on an electrode. The negative control probe was a nucleotide having a sequence completely unrelated to the SAA1 gene.

**Table 4 Nucleotide probe used for detection of the product amplified with C-13T detecting primer set 2**

| | | SEQ ID No. | Base number | Tm value | F/R | Sequence |
|---|---|---|---|---|---|---|
| | Negative control | 26 | 14mer | | | GTGCTGCAGGTGCG |
| C-13T | Wild-type | 27 | 14mer | 63.0 | F | CACCGCTCCCTGGC |
| | | 28 | 16mer | 70.2 | F | CCACCGCTCCCTGGCA |
| | | 29 | 17mer | 73.7 | F | GCCACCGCTCCCTGGCA |
| | | 30 | 18mer | 74.0 | F | AGCCACCGCTCCCTGGCA |
| | | 31 | 20mer | 76.6 | F | CAGCCACCGCTCCCTGGCAG |
| | Variant | 32 | 15mer | 63.3 | F | CACCGTTCCCTGGCA |
| | | 33 | 17mer | 68.1 | F | CCACCGTTCCCTGGCAG |
| | | 34 | 18mer | 71.5 | F | AGCCACCGTTCCCTGGCA |
| | | 35 | 19mer | 74.1 | F | CAGCCACCGTTCCCTGGCA |
| | | 36 | 20mer | 74.4 | F | CAGCCACCGTTCCCTGGCAG |

Nucleotide probe-immobilized support:

Gold electrodes were made on a DNA chip, and the nucleotide probes were immobilized thereon. Immobilization was performed by using the strong bonding force between thiol and gold. A probe solution containing a nucleotide probe with a thiol-modified terminal was spotted on the gold electrode, and, after 1 hour, the DNA chip was immersed in 1 mM mercaptohexanol solution and then, washed with 0.2xSSC solution. The same probe was spotted on two electrodes. After washing, the chip was washed with ultrapure water and dried in air, to give a nucleotide probe-immobilized support.

The nucleotide probes were immobilized on the following electrodes respectively:
Electrode 1-2: negative probe (SEQ ID No. 26),
Electrode 3-4: wild-type nucleotide probe 14mer (SEQ ID No. 27),
Electrode 5-6: wild-type nucleotide probe 16mer (SEQ ID No. 28),
Electrode 7-8: wild-type nucleotide probe 17mer (SEQ ID No. 29),
Electrode 9-10: wild-type nucleotide probe 18mer (SEQ ID No. 30),
Electrode 11-12: wild-type nucleotide probe 20mer (SEQ ID No. 31),
Electrode 13-14: variant nucleotide probe 15mer (SEQ ID No. 32),
Electrode 15-16: variant nucleotide probe 17mer (SEQ ID No. 33),
Electrode 17-18: variant nucleotide probe 18mer (SEQ ID No. 34),
Electrode 19-20: variant nucleotide probe 19mer (SEQ ID No. 35), and
Electrode 21-22: variant nucleotide probe 20mer (SEQ ID No. 36).

Hybridization between amplification products and nucleotide probe, and detection thereof:

To the amplification products obtained by amplification were added salts at a final concentration of 2xSSC, and the mixture was allowed to hybridize with the electrode-immobilized nucleotide probe. The reaction temperatures were 35, 45, 50, 55, and 60°C, and the reaction period was 60 minutes. Then, the DNA chip was washed mildly with ultrapure water. The DNA chip was immersed in a phosphate buffer containing 50 µM of an intercalating agent Hoechst 33258 for 10 minutes and washed, and then the oxidative current response of the Hoechst 33258 molecule was measured.

### [Test 3-1: Results]

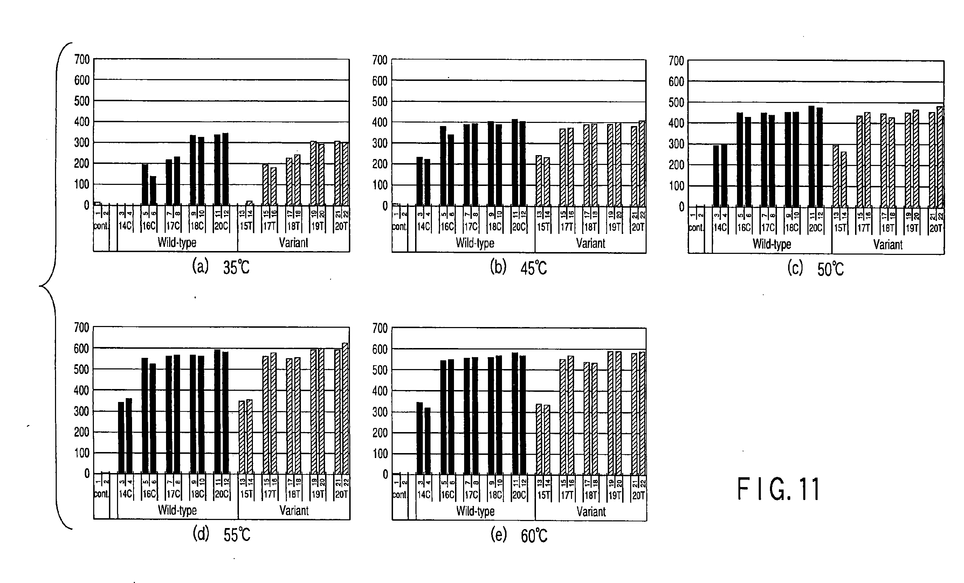

The results are summarized in FIG. 11. The signal increased as the reaction temperature increased, indicating that an increase in reaction temperature leads to an increase in hybridization efficiency. The tests at reaction temperatures of 55 and 60°C gave almost the same results.

### [Test 3-2: Probe for detection of C-13T]

Then, hybridization was performed in a similar manner to the test 3-1, except that the reaction temperature was 55°C and the reaction times were 10, 20, 40, 60, and 120 minutes.

### [Test 3-2: Result]

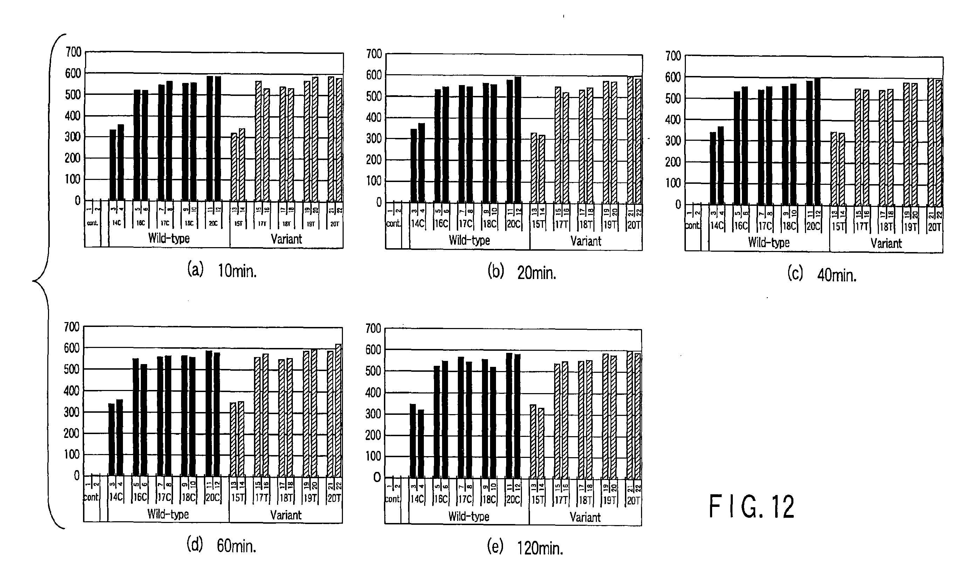

The results are summarized in FIG. 12. The tests at reaction times of 10 and 120 minutes gave almost the same results, indicating that the hybridization reaction was already in the saturated state after 10 minutes.

Wild-type nucleotide probe (14C: SEQ ID No. 27) and variant nucleotide probes (15T: SEQ ID No. 32) relatively shorter in chain length gave a signal relatively lower in intensity. Wild-type nucleotide probes (16C: SEQ ID No. 28, 17C: SEQ ID No. 29, 18C: SEQ ID No. 30, and 20C: SEQ ID No. 31) and variant nucleotide probes (17T: SEQ ID No. 33, 18T: SEQ ID No. 34, 19T: SEQ ID No. 35, and 20T: SEQ ID No. 36) gave a signal almost the same in intensity.

### [Test 3-3: Probe for detection of C-13T]

Then, hybridization was performed in a manner similar to the test 3-1 at a reaction temperature of 55°C for 20 minutes. Subsequently, the nucleotide probe-immobilized support was immersed for 20 minutes in a 0.2xSSC washing buffer at 40, 45, or 50°C. The analyte nucleotides used for amplification in the present test were 3 kinds of human genomes determined to be respectively wild homozygous, variant homozygous, and heterozygous respectively, by PCR-RFLP analysis.

### [Test 3-3: Results]

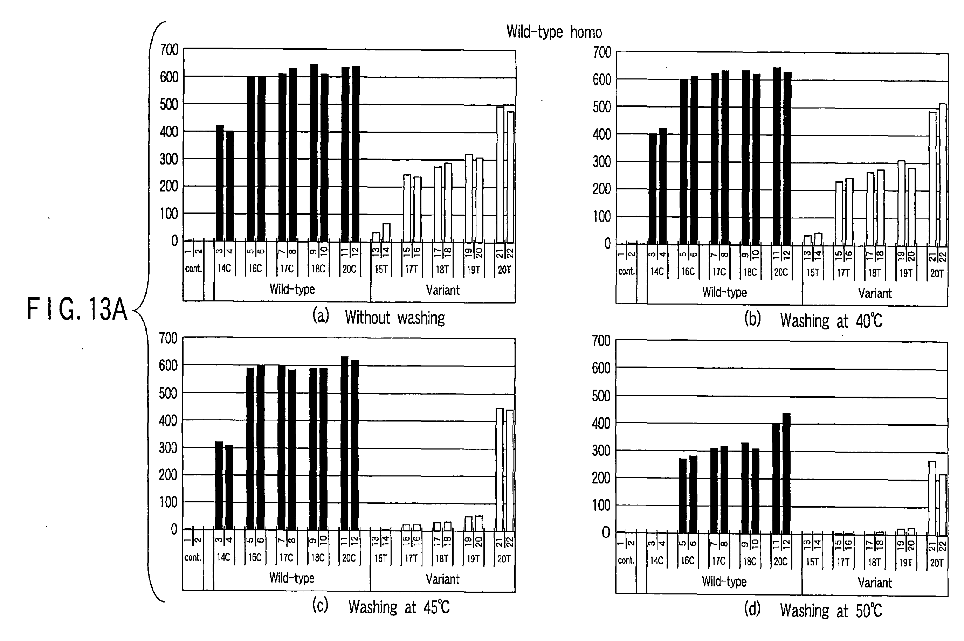

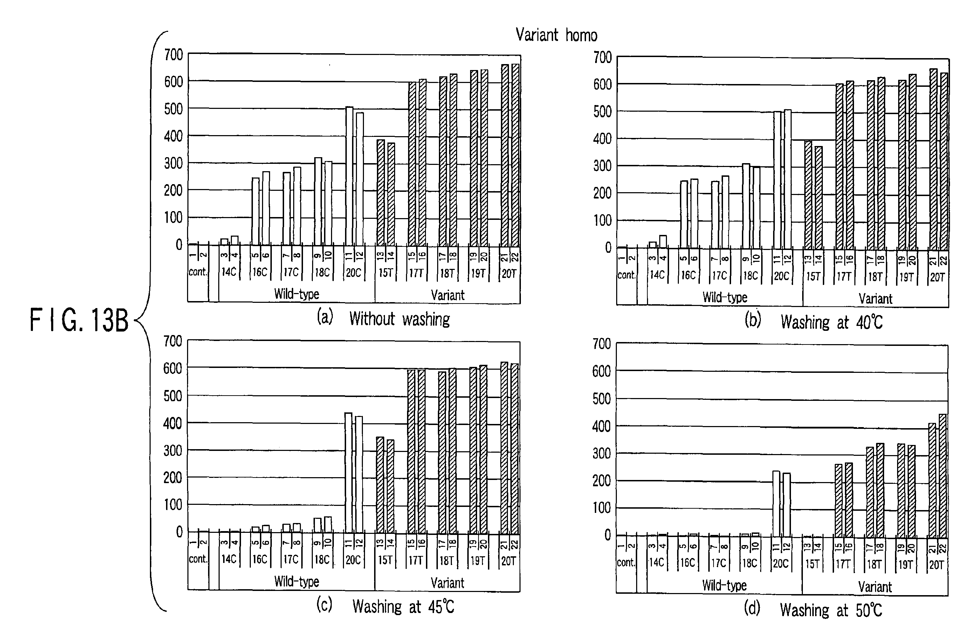

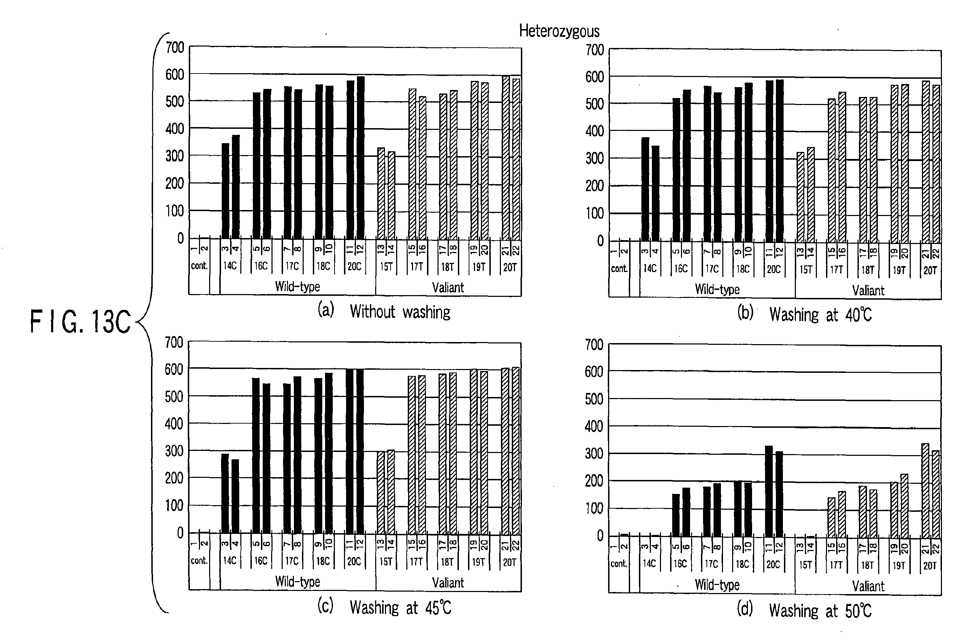

The results are summarized in FIGS. 13A to 13C. When the DNA chip was not washed (only washed mildly with ultrapure water), there were wild-type amplification products detected by the variant nucleotide probe and also variant amplification products detected by the wild-type nucleotide probe, indicating the presence of non-specific hybridization. The results at a washing temperature of 40°C were almost the same as those without washing.

At a washing temperature of 45°C, wild-type amplification products were detected by wild-type nucleotide probes (16C, 17C, and 18C) at high signal intensity, but almost no signal was detected by a variant nucleotide probe (17T, 18T, and 19T). Similarly, variant amplification products were detected by the variant nucleotide probe (17T, 18T, and 19T) at high signal intensity, but almost no signal was detected by the wild-type nucleotide probes (16C, 17C, and 18C), indicating that bonds formed by non-specific hybridization were broken by washing at 45°C. Alternatively, heterozygous amplification products were detected both by the wild-type nucleotide probes (16C, 17C, and 18C) and the variant nucleotide probe (17T, 18T, and 19T) at high signal intensity.

At a washing temperature of 50°C, the current detected was lower, indicating that amplification products were separated from the nucleotide probe by washing. At a washing temperature of 50°C, If the amplification product is the wild-type, although the signal by the wild-type nucleotide probe decreased, the signal by the variant nucleotide probe (20T) remained high. Similarly, If the amplification product is the variant, although the signal by the variant nucleotide probe decreased, the signal by the wild-type nucleotide probe (20C) remained high, indicating that there was still some non-specific binding although the specific signal was reduced.

The results showed that the wild-type nucleotide probes (16C, 17C, and 18C) and the variant nucleotide probe (17T, 18T, and 19T) give an ideal detection pattern. Accordingly, the nucleotide probes most preferably used according to the present invention are the wild-type nucleotide probes (16C: SEQ ID No. 28, 17C: SEQ ID No. 29, and 18C: SEQ ID No. 30) and the variant nucleotide probe (17T: SEQ ID No. 33, 18T: SEQ ID No. 34, and 19T: SEQ ID No. 35).

The Tm values of the nucleotide probes used in the test are also summarized in Table 4. As apparent from Table 4, the nucleotide probes preferably used in the present invention are wild-type nucleotide probes having a Tm value of 63 to 77°C, preferably 70 to 74°C, and variant nucleotide probes having a Tm value of 63 to 74°C, preferably 68 to 74°C.

### [Test 4-1: Probe for detection of C2995T]

LAMP amplification was performed at 63°C for 1 hour by using a human genome determined to be heterozygous by PCR-RFLP analysis as the template and also by using the primer set 3 for detection of C2995T and T3010C. The primer set 3 for detection of C2995T and T3010C were the sets determined to be the best in the test 2 above. The amplification products obtained were detected on a current-detection DNA chip.

### Nucleotide probe:

The nucleotide sequences of the nucleotide probes tested are summarized in Table 5. The nucleotide probe used was a plus chain. The 3' terminal of the nucleotide probe was thiol-modified for immobilization on an electrode. The negative control probe was a nucleotide having a sequence completely unrelated to the SAA1 gene sequence.

**Table 5 Nucleotide probe for C-2995T used for detection of the product amplified with C-2995T and T3010C detecting primer set 3**

| | | SEQ ID No. | Base number | Tm value | F/R | Sequence |
|---|---|---|---|---|---|---|
| C2995T | Wild-type | 37 | 13 | 62.6 | F | GGGGTGCCTGGGC |
| | | 38 | 14 | 66.9 | F | GGGGGTGCCTGGGC |
| | | 39 | 15 | 70.1 | F | TGGGGGTGCCTGGGC |
| | | 40 | 16 | 70.7 | F | CTGGGGGTGCCTGGGC |
| | | 41 | 17 | 73.8 | F | CCTGGGGGTGCCTGGGC |
| | Variant | 42 | 14 | 61.2 | F | GGGGGTGTCTGGGC |
| | | 43 | 16 | 65.7 | F | CTGGGGGTGTCTGGGC |
| | | 44 | 17 | 69.4 | F | CCTGGGGGTGTCTGGGC |
| | | 45 | 18 | 69.9 | F | ACCTGGGGGTGTCTGGGC |
| | | 46 | 20 | 74.3 | F | GGACCTGGGGGTGTCTGGGC |

Nucleotide probe-immobilized support:

A nucleotide probe-immobilized support was prepared in a similar manner to the test 3-1.

The nucleotide probes were immobilized on the following electrodes respectively:
Electrode 1-2: negative probe (SEQ ID No. 26),
Electrode 3-4: wild-type nucleotide probe 13mer (SEQ ID No. 37),
Electrode 5-6: wild-type nucleotide probe 14mer (SEQ ID No. 38),
Electrode 7-8: wild-type nucleotide probe 15mer (SEQ ID No. 39),
Electrode 9-10: wild-type nucleotide probe 16mer (SEQ ID No. 40),
Electrode 11-12: wild-type nucleotide probe 17mer (SEQ ID No. 41),
Electrode 13-14: variant nucleotide probe 14mer (SEQ ID No. 42),
Electrode 15-16: variant nucleotide probe 16mer (SEQ ID No. 43),
Electrode 17-18: variant nucleotide probe 17mer (SEQ ID No. 44),
Electrode 19-20: variant nucleotide probe 18mer (SEQ ID No. 45), and
Electrode 21-22: variant nucleotide probe 20mer (SEQ ID No. 46).

Hybridization between amplification products and nucleotide probe, and detection thereof:

To the amplification products obtained by amplification were added salts at a final concentration of 2xSSC, and the mixture was allowed to hybridize with the electrode-immobilized nucleotide probe. The reaction temperatures were 35, 45, 50, 55, and 60°C, and the reaction period was 20 minutes. Then, the DNA chip was washed mildly with ultrapure water. The DNA chip was immersed in a phosphate buffer containing 50 µM of an intercalating agent Hoechst 33258 for 10 minutes and washed, and then, the oxidative current response of the Hoechst 33258 molecule was measured.

### [Test 4-1: Results]

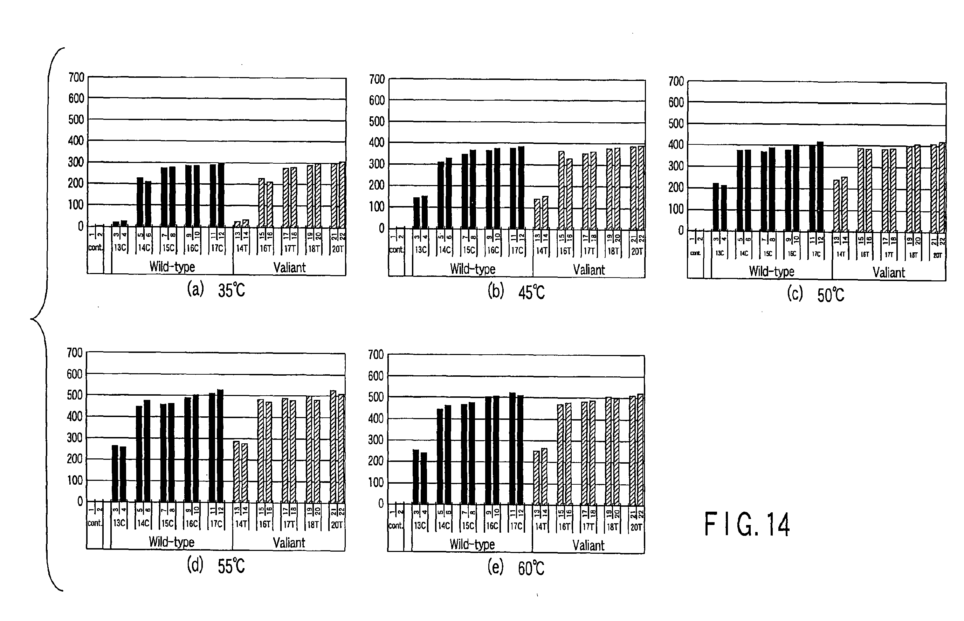

The results are summarized in FIG. 14. The signal increased as the reaction temperature increased, indicating that an increase in reaction temperature leads to an increase in hybridization efficiency. The tests at reaction temperatures of 55°C and 60°C gave almost the same results.

### [Test 4-2: Probe for detection of C2995T]

Then, hybridization was performed in a manner similar to the test 3-3 at a reaction temperature of 55°C for 20 minutes by using the products amplified with the primer set 3. Subsequently, the nucleotide probe-immobilized support was washed as immersed in a 0.2xSSC washing buffer at 45°C for 20 minutes. The analyte nucleotides used for amplification in the present test were 3 kinds of human genomes determined to be wild homozygous, variant homozygous and heterozygous respectively, by PCR-RFLP analysis. The nucleotide probes and the detection method used in the test were the same as those for the test 4-1.

### [Test 4-2: Results]

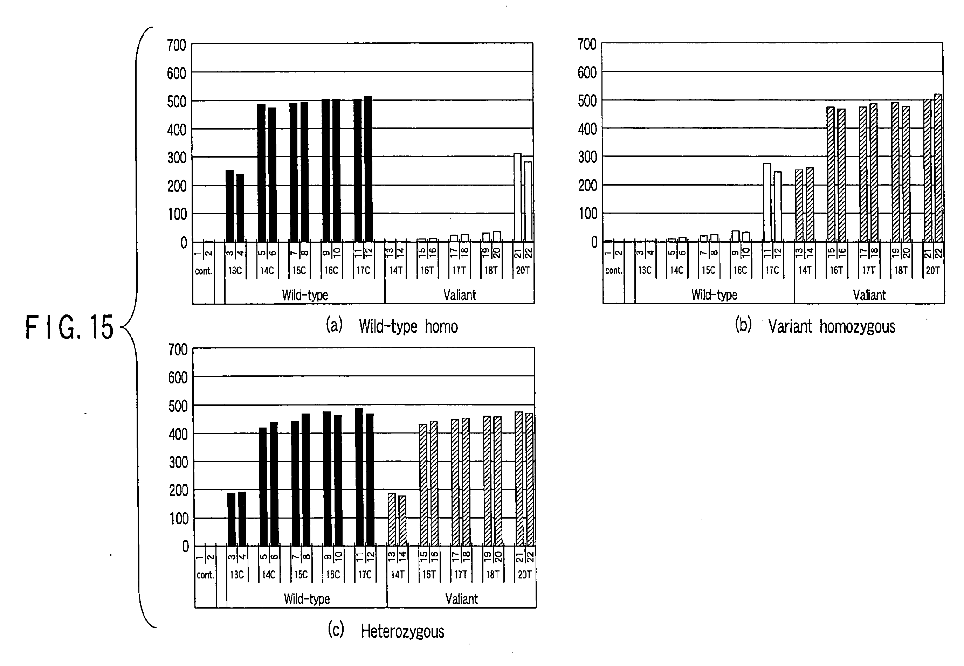

The results are summarized in FIG. 15. Wild-type amplification products were detected by wild-type nucleotide probes (14C, 15C, and 16C) at high signal intensity, while almost no signal was detected by the variant nucleotide probes (16T, 17T, and 18T). Similarly, variant amplification products were detected by the variant nucleotide probes (16T, 17T, and 18T) at high signal intensity, while almost no signal was detected by the wild-type nucleotide probes (14C, 15C, and 16C). In addition, heterozygous amplification products were detected both by the wild-type nucleotide probes (14C, 15C, and 16C) and also by the variant nucleotide probes (16T, 17T, and 18T) at high signal intensity.

The results showed that the wild-type nucleotide probes (14C, 15C, and 16C) and the variant nucleotide probes (16T, 17T, and 18T) give an ideal detection pattern. Thus, the nucleotide probes used most preferably according to the present invention are the wild-type nucleotide probes (14C: SEQ ID No. 38, 15C: SEQ ID No. 39, and 16C: SEQ ID No. 40) and the variant nucleotide probes (16T: SEQ ID No. 43, 17T: SEQ ID No. 44, and 18T: SEQ ID No. 45).

The Tm values of the nucleotide probes used were also summarized in Table 5. As apparent from in Table 5, the nucleotide probes preferably used in the present invention are wild-type nucleotide probes having a Tm value of 63 to 74°C, preferably 67 to 71°C, and variant nucleotide probes having a Tm value of 61 to 74°C, preferably 66 to 70°C.

### [Test 5: Probe for detection of T3010C]

LAMP amplification was performed at 63°C for 1 hour, by using human genomes determined to be wild homozygous, variant homozygous, and heterozygous by PCR-RFLP analysis as the templates and the primer set 3 for detection of C2995T and T3010C. The primer set 3 for detection of C2995T and T3010C was the sets determined to be the best in the test 2 above. The amplification products obtained were detected on a current-detection DNA chip.

### Nucleotide probe:

The nucleotide sequences of the nucleotide probes tested are summarized in Table 6. The nucleotide probes used was a plus chain. The 3' terminal of the nucleotide probe was thiol-modified for immobilization on an electrode. The negative control probe used was a nucleotide having a sequence completely unrelated to the SAA1 gene sequence.

**Table 6 Nucleotide probe for T3010C used for detection of the product amplified with C-2995T and T3010C detecting primer set 3**

| | | SEQ ID No. | Base number | Tm value | F/R | Sequence |
|---|---|---|---|---|---|---|
| T3010C | Wild-type | 47 | 19 | 54.5 | R | GTTACCTGATCACTTCTGC |
| | | 48 | 21 | 58.9 | R | CAGTTACCTGATCACTTCTGC |
| | | 49 | 22 | 62.6 | R | CCAGTTACCTGATCACTTCTGC |
| | | 50 | 23 | 64.3 | R | TCCAGTTACCTGATCACTTCTGC |
| | | 51 | 26 | 70.2 | R | TCCAGTTACCTGATCACTTCTGCRGC |
| | Variant | 52 | 17 | 54.4 | R | TTACCTGATCGCTTCTG |
| | | 53 | 19 | 60.0 | R | GTTACCTGATCGCTTCTGC |
| | | 54 | 20 | 61.0 | R | AGTTACCTGATCGCTTCTGC |
| | | 55 | 21 | 63.8 | R | CAGTTACCTGATCGCTTCTGC |
| | | 56 | 22 | 66.9 | R | CCAGTTACCTGATCGCTTCTGC |
| | | 57 | 25 | 71.1 | R | TCCAGTTACCTGATCGCTTCTGCRG |

Nucleotide probe-immobilized support:

A nucleotide probe-immobilized support was prepared in a similar manner to the test 3-1.

The nucleotide probes were immobilized on the following electrodes respectively:
Electrode 1-2: negative probe (SEQ ID No. 26),
Electrode 3-4: wild-type nucleotide probe 19mer (SEQ ID No. 47),
Electrode 5-6: wild-type nucleotide probe 21mer (SEQ ID No. 48),
Electrode 7-8: wild-type nucleotide probe 22mer (SEQ ID No. 49),
Electrode 9-10: wild-type nucleotide probe 23mer (SEQ ID No. 50),
Electrode 11-12: wild-type nucleotide probe 26mer (SEQ ID No. 51),
Electrode 13-14: variant nucleotide probe 17mer (SEQ ID No. 52),
Electrode 15-16: variant nucleotide probe 19mer (SEQ ID No. 53),
Electrode 17-18: variant nucleotide probe 20mer (SEQ ID No. 54),
Electrode 19-20: variant nucleotide probe 21mer (SEQ ID No. 55),
Electrode 21-22: variant nucleotide probe 22mer (SEQ ID No. 56), and
Electrode 23-24: variant nucleotide probe 25mer (SEQ ID No. 57).

Hybridization between amplification products and nucleotide probe, and detection thereof:

To the amplification products obtained by amplification were added salts at a final concentration of 2xSSC, and the mixture was allowed to hybridize with the electrode-immobilized nucleotide probe. The reaction temperature was 55°C, and the reaction period was 20 minutes. Then, the chip was washed at 45°C for 20 minutes. The electrode was immersed in a phosphate buffer containing 50 µM of an intercalating agent Hoechst 33258 for 10 minutes and washed, and then the oxidative current response of the Hoechst 33258 molecule was measured.

### [Test 5: Result]

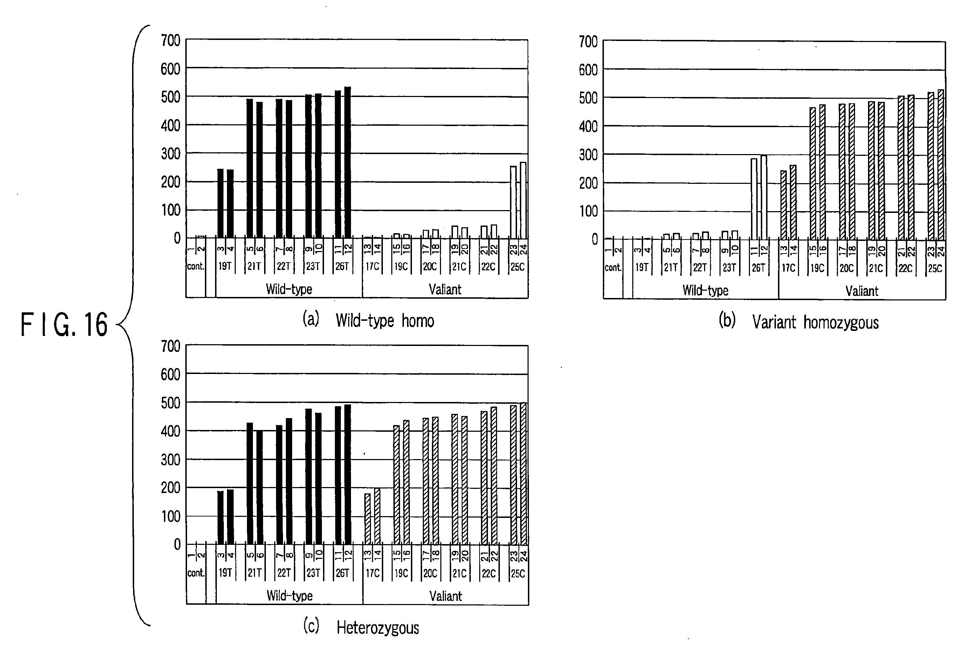

The results are summarized in FIG. 16. Nucleotide probes for detection of wild-type mutation (21T, 22T, and 23T) and nucleotide probes for detection of variants (19C, 20C, 21C, and 22C) showed an ideal detection pattern. Wild-type amplification products were detected by the wild-type nucleotide probes (21T, 22T, and 23T) at high signal intensity, while almost no signal increase was detected by the variant nucleotide probes (19C, 20C, 21C, and 22C) in which non-specific hybridization was broken. Similarly, variant amplification products were detected by the variant nucleotide probes (19C, 20C, 21C, and 22C) at high signal intensity, while almost no signal increase was detected by the wild-type nucleotide probes (21T, 22T, and 23T) in which non-specific hybridization was broken. In addition, heterozygous amplification products were detected both by the wild-type nucleotide probes (21T, 22T, and 23T) and the variant nucleotide probes (19C, 20C, 21C, and 22C) at high signal intensity.

The results showed that the nucleotide probes used most preferably according to the present invention were the wild-type nucleotide probes (21T: SEQ ID No. 48, 22T: SEQ ID No. 49, and 23T: SEQ ID No. 50) and the variant nucleotide probes (19C: SEQ ID No. 53, 20C: SEQ ID No. 54, 21C: SEQ ID No. 55, and 22C: SEQ ID No. 56).

The Tm values of the nucleotide probes used in the test are also summarized in Table 6. As apparent from the results in Table 6, the nucleotide probes preferably used in the present invention are wild-type nucleotide probes having a Tm value of 55 to 70°C, preferably 59 to 64°C, and variant nucleotide probes having a Tm value of 55 to 71°C, preferably 60 to 67°C.

### <Analyte Sample>

The analyte sample subjected to the present invention is not particularly limited, and may be, for example, human blood, serum, leukocyte, hair root, or oral mucous membranes. Nucleotide components are extracted from the analyte sample, to prepare a sample solution subjected to the test for detecting a target nucleotide. The extraction method is not particularly limited, and, for example, a commercially available nucleotide extraction tool such as QIAamp (manufactured by QIAGEN) or Sumai test (Sumitomo Metal Industries, Ltd.) may be used.

### <Kit>

Another aspect of the present invention provides a kit including the primer set described above for the LAMP method for use in the detection method according to the present invention. The kit may optionally contain, for example, a chain-substituting DNA polymerase, a synthesis substrate, and a buffer solution.

In another aspect, the invention provides a nucleotide probe for detection of the amplification products amplified with the primer set according to the inbention. The present invention also provides a nucleotide probe-immobilized support on which the nucleotide probe according to the invention was immobilized. The probe-immobilized support is provided preferably as a DNA chip or DNA microarray.

The kit according to the invention can also included the nucleotide probe or the nucleotide probe-immobilized support additionally.

In another aspect, the present invention, provided is a method of detecting single-nucleotide polymorphisms C-13T, C2995T and T3010C of SAA1 simultaneously. In the present aspect, C-13T, C2995T and T3010C are amplified separately as described above, and then the amplification products are mixed to prepare a liquid mixture. The liquid mixture is subjected to the detection as described above. In the present aspect, it is possible to detect the genotypes of C-13T, C2995T and T3010C simultaneously.

### EXAMPLES

### [Simultaneous detection of C-13T, C2995T, and T3010C]

A test for simultaneous detection of C-13T, C2995T, and T3010C was performed. A human genome was amplified at 63°C for 1 hour, respectively by using the nucleotide primer set 2 for detection of C-13T (test 1) and the nucleotide primer set 3 for detection of C2995T and T3010C (test 2). The human genomes used were three kinds of human genomes determined to be wild homozygous, variant homozygous and heterozygous respectively by PCR-RFLP analysis. After the amplification reaction, two kinds of amplification products were mixed, to prepare a mixed reaction solution.

### <Shortening of amplification period by introduction of loop primer>

The nucleotide primer set 2 for detection of C-13T in a 25 µl reaction solution was added 40 pmol of an LFc loop primer of SEQ ID No. 58. The nucleotide primer set 3 for detection of C2995T and T3010C in a 25 µl reaction solution was added 40 pmol of an LBc loop primer of SEQ ID No. 59. Then, the mixture was amplified respectively. As a result, the amplification period required for saturation was shortened from 60 minutes to about 30 minutes in both cases. The LAMP amplification product for C-13T detection and that for detection of C2995T and T3010C were mixed, and the mixture was subjected to simultaneous detection of C-13T, C2995T, and T3010C.

### Nucleotide probe:

The mixed reaction solution was subjected to the detection with the nucleotide probe for C-13T, the nucleotide probe for C2995, and the nucleotide probe for T3010C.

The nucleotide probes were as follows:
C-13T wild-type nucleotide probe (SEQ ID No. 28), variant nucleotide probe SEQ ID No. 33);
C2995T wild-type nucleotide probe (SEQ ID No. 38), variant nucleotide probe (SEQ ID No. 43); and
T3010C wild-type nucleotide probe (SEQ ID No. 50), variant nucleotide probe (SEQ ID No. 55).

The nucleotide probe for C-13T and C2995T was a plus-chain probe, while that for T3010C, a minus-chain probe. The 3' terminals of all nucleotide probes were thiol-modified.

Preparation of probe nucleotide-immobilized electrode:

A probe nucleotide-immobilized electrode was prepared in a manner similar to the method described in the test 3-1 above.

The nucleotide probes were immobilized on the following electrodes respectively:
Electrode 1-2: negative probe (SEQ ID No. 26),
Electrode 3-4: C-13T wild-type nucleotide probe 16mer (SEQ ID No. 28),
Electrode 5-6: C-13T variant nucleotide probe 17mer (SEQ ID No. 33),
Electrode 7-8: C2995T wild-type nucleotide probe 14mer (SEQ ID No. 38),
Electrode 9-10: C2995T variant nucleotide probe 16mer (SEQ ID No. 43),
Electrode 11-12: T3010C variant nucleotide probe 23mer (SEQ ID No. 50), and
Electrode 13-14: T3010C variant nucleotide probe 21mer (SEQ ID No. 55).

Hybridization between amplification products and nucleotide probe, and detection thereof:

Hybridization was performed similarly to the test 3 at 55°C for 20 minutes and cleaning at 45°C for 20 minutes, and then, the DNA chip was washed lightly with ultrapure water. The electrode was immersed for 10 minutes in a phosphate buffer containing an 50 µM Hoechst 33258 solution as intercalating agent, and then, the oxidative current response of the Hoechst 33258 molecule was determined.

### [Results]

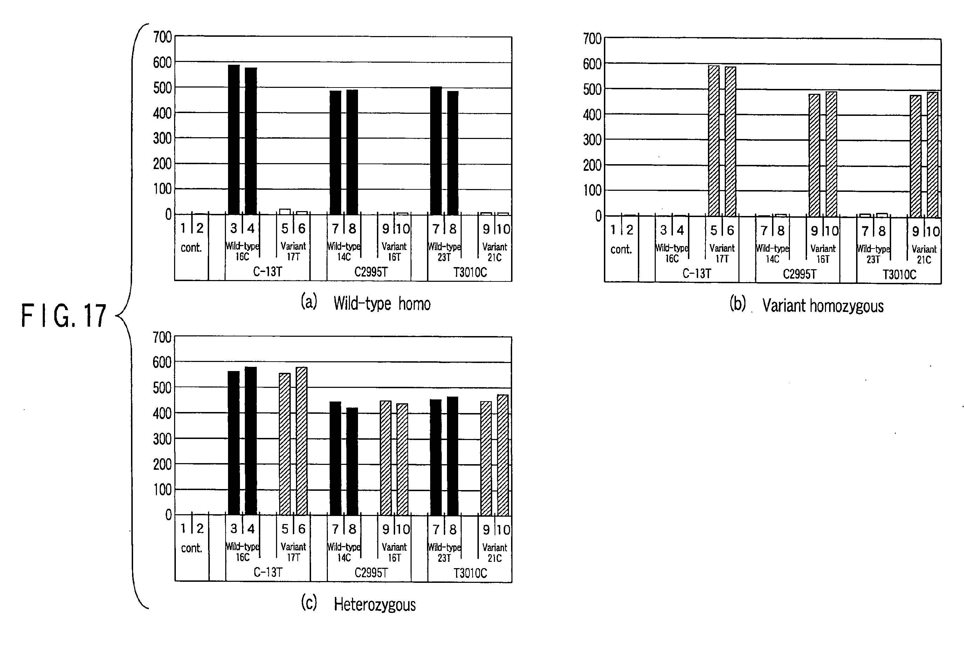

Results are summarized in FIG. 17. An ideal detection pattern was observed for C-13T, C2995T, and T3010C. The results showed that it was possible to detect C-13T, C2995T, and T3010C simultaneously by using a DNA chip.

## Claims

1. A nucleotide primer set for LAMP amplification, used for detecting a genotype of the SAA1 gene single-nucleotide polymorphism C-13T, **characterized in that**
when a target nucleotide has an F3 region, F2 region and F1 region in turn from a 5' terminal and B3c region, B2c region and B1c region in turn from a 3'-terminal, and
when there are nucleotide primers including an FIP primer having a sequence identical with that of the F2 region in the 3'-terminal side and a sequence complementary to the F1 region in the 5'-terminal side, an F3 primer having a sequence identical with that of the F3 region, a BIP primer having a sequence complementary to the B2c region in the 3'-terminal side and a sequence identical with that of the B1c region in the 5' terminal side, and a B3 primer having a sequence complementary to the B3c region,
the primer set comprising:
an FIP primer and a BIP primer selected from the primer sets 1 to 7 shown in Table 2;
an F3 primer binding to a region within 60 bases from the 5' terminal of the F2 region of the target nucleic acid; and
a B3 primer binding to a region within 60 bases from the 3' terminal of the B2c region of the target nucleic acid.

2. The nucleotide primer set according to claim 1, **characterized in that** the FIP and BIP primers are selected from the primer sets 1, 2, 3, 4, and 6 shown in Table 2.

3. The nucleotide primer set according to claim 1, **characterized in that** the FIP and BIP primers are the primers of the primer set 2 shown in Table 2.

4. A method of detecting a genotype of a single-nucleotide polymorphism C-13T of the SAA1 gene, **characterized by** comprising the steps of:
obtaining an amplification product by amplification of a target nucleic acid by using the nucleotide primer set according to claim 1; and
measuring and comparing the amounts of the wild-type amplification product and the variant amplification product contained in the amplification product.

5. The method according to claim 4, **characterized in that**
the amplification product is measured by hybridization of nucleotide probes immobilized on a support (1, 11) with the amplification product and subsequent determination of an amounts of the amplification product bound to the nucleotide probe, and
the nucleotide probes include a wild-type nucleotide probe complementary to the wild-type amplification product and a variant nucleotide probe complementary to the variant amplification product.

6. The method according to claim 5, **characterized in that** the wild-type nucleotide probe has a Tm value of 63 to 77°C, the variant nucleotide probe has a Tm value of 63 to 74°C, and the single nucleotide polymorphism C-13T site is located inside by three or more bases from the terminal of the respective nucleotide probes.

7. The method according to claim 6, **characterized in that** the wild-type nucleotide probe has a Tm value of 70 to 74°C and the variant nucleotide probe has a Tm value of 68 to 74°C.

8. The method according to claim 5, **characterized in that** the wild-type nucleotide probe has a sequence of SEQ ID No. 28, 29 or 30, or a sequence complementary thereto, and the variant nucleotide probe has a sequence of SEQ ID No. 33, 34 or 35, or a sequence complementary thereto.

9. The method according to claim 8, **characterized in that** the wild-type nucleotide probe has a sequence of SEQ ID No. 28 or a sequence complementary thereto, and the variant nucleotide probe has a sequence of SEQ ID No. 33 or a sequence complementary thereto.

10. A nucleotide probe for detecting a genotype of a single-nucleotide polymorphism C-13T of a SAA1 gene from an amplification product obtained by amplification of a target nucleotide by using a nucleotide primer set,
**characterized by** comprising:
a nucleotide probe selected from a wild-type nucleotide probe and a variant nucleotide probe,
wherein
the wild-type nucleotide probe is complementary to the wild-type amplification product and has a Tm value of 63 to 77°C, and the single-nucleotide polymorphism C-13T site is located inside by three or more bases from the terminal thereof, and
the variant nucleotide probe is complementary to the variant amplification product and has a Tm value of 63 to 74°C, and the single-nucleotide polymorphism C-13T site is located inside by three or more bases from the terminal thereof; and wherein
when a target nucleotide has F3 region, F2 region and F1 region in turn from a 5' terminal and B3c region, B2c region and B1c region in turn from a 3'-terminal,
when the nucleotide primer set includes an FIP primer having a sequence identical with that of the F2 region in the 3'-terminal side and a sequence complementary to the F1 region in the 5'-terminal side, an F3 primer having a sequence identical with that of the F3 region, a BIP primer having a sequence complementary to the B2c region in the 3'-terminal side and a sequence identical with that of the B1c region in the 5' terminal side, and a B3 primer having a sequence complementary to the B3c region,
the FIP and BIP primers are selected from the primer sets 1 to 7 shown in Table 2;
the F3 primer binds to a region within 60 bases from the 5' terminal of the F2 region of the target nucleic acid; and
the B3 primer binds to a region within 60 bases from the 3' terminal of the B2c region of the target nucleic acid.

11. The nucleotide probe according to claim 10, **characterized in that** the wild-type nucleotide probe has a sequence of SEQ ID No. 28, 29 or 30, or a sequence complementary thereto, and the variant nucleotide probe has a sequence of SEQ ID No. 33, 34 or 35, or a sequence complementary thereto.

12. The nucleotide probe according to claim 10, **characterized in that** the probes are immobilized on a support (1, 11).

13. A nucleotide primer set for LAMP amplification, used for detecting a genotype of a single nucleotide polymorphism C2995T and/or 3010C of the SAA1 gene, **characterized in that**
when a target nucleotide has an F3 region, F2 region and F1 region in turn from a 5' terminal and B3c region, B2c region and B1c region in turn from a 3'-terminal, and
when there are nucleotide primers including an FIP primer having a sequence identical with that of the F2 region in the 3'-terminal side and a sequence complementary to the F1 region in the 5'-terminal side, an F3 primer having a sequence identical with that of the F3 region, a BIP primer having a sequence complementary to the B2c region in the 3'-terminal side and a sequence identical with that of the B1c region in the 5' terminal side, and a B3 primer having a sequence complementary to the B3c region,
the primer set comprising:
an FIP primer and a BIP primer selected from the primer sets 1 to 4 shown in Table 3;
an F3 primer binding to a region within 60 bases from the 5' terminal of the F2 region of the target nucleic acid; and
a B3 primer binding to a region within 60 bases from the 3' terminal of the B2c region of the target nucleic acid.

14. The nucleotide primer set according to claim 13, **characterized in that** the FIP and BIP primers are primers of the primer set 3 shown in Table 3.

15. A method of detecting a genotype of a single-nucleotide polymorphism C2995T or T3010C of the SAA1 gene, **characterized by** comprising the steps of:
obtaining an amplification product by amplifying a target nucleic acid by using the nucleotide primer set according to claim 13; and
measuring and comparing amounts of a wild-type amplification product and a variant amplification product contained in the amplification product.

16. The method according to claim 15, **characterized in that**
the amplification product is measured by hybridization of nucleotide probes immobilized on a support (1, 11) with the amplification product and subsequent determination of an amount of the amplification product bound to the nucleotide probe, and
the nucleotide probes include a wild-type nucleotide probe complementary to the wild-type amplification product and a variant nucleotide probe complementary to the variant amplification product.

17. The method according to claim 16 for detection of the genotype of the single nucleotide polymorphism C2995T, **characterized in that** the wild-type nucleotide probe has a Tm value of 63 to 74°C, the variant nucleotide probe has a Tm value of 61 to 74°C, and the single-nucleotide polymorphism C2995T site is located inside by three or more bases from the terminal of the respective nucleotide probes.

18. The method according to claim 17, **characterized in that** the wild-type nucleotide probe has a Tm value of 67 to 71°C, and the variant nucleotide probe has a Tm value of 66 to 70°C.

19. The method according to claim 18, **characterized in that** the wild-type nucleotide probe has a sequence of SEQ ID No. 38, 39 or 40, or a sequence complementary thereto, and the variant nucleotide probe has a sequence of SEQ ID No. 43, 44 or 45 or a sequence complementary thereto.

20. The method according to claim 19, **characterized in that** the wild-type nucleotide probe has a sequence of SEQ ID No. 38 or a sequence complementary thereto, and the variant nucleotide probe has a sequence of SEQ ID No. 43 or a sequence complementary thereto.

21. The method according to claim 16 for detection of the genotype of the single nucleotide polymorphism T3010C, **characterized in that** the wild-type nucleotide probe has a Tm value of 55 to 70°C, the variant nucleotide probe has a Tm value of 55 to 71°C, and the single-nucleotide polymorphism T3010C site is located inside by three or more bases from the terminal of respective nucleotide probes.

22. The method according to claim 21, **characterized in that** the wild-type nucleotide probe has a Tm value of 59 to 64°C, and the variant nucleotide probe has a Tm value of 60 to 67°C.

23. The method according to claim 22, **characterized in that** the wild-type nucleotide probe has a sequence of SEQ ID No. 48, 49 or 50, or a sequence complementary thereto, and the variant nucleotide probe has a sequence of SEQ ID No. 53, 54, 55 or 56, or a sequence complementary thereto.

24. The method according to claim 23, **characterized in that** the wild-type nucleotide probe has the sequence of SEQ ID No. 50 or a sequence complementary thereto, and the variant nucleotide probe has the sequence of SEQ ID No. 55 or a sequence complementary thereto.

25. A nucleotide probes for detecting a genotype of a single-nucleotide polymorphism C2995T of the SAA1 gene from an amplification product obtained by amplification of a target nucleotide by using a nucleotide primer set,
**characterized by** comprising:
a nucleotide probe selected from a wild-type nucleotide probe and a variant nucleotide probe,
wherein
the wild-type nucleotide probe is complementary to the wild-type amplification product and has a Tm value of 63 to 74°C, and the single-nucleotide polymorphism C2995T site is located inside by three or more bases from the terminal thereof, and
the variant nucleotide probe is complementary to the variant amplification product and has a Tm value of 61 to 74°C, and the single-nucleotide polymorphism C2995T site is located inside by three or more bases from the terminal thereof; and wherein
when the target nucleotide has F3 region, F2 region and F1 region in turn from a 5' terminal and B3c region, B2c region and B1c region in turn from a 3'-terminal,
when the nucleotide primer set includes an FIP primer having a sequence identical with that of the F2 region in the 3' terminal side and a sequence complementary to the F1 region in the 5' terminal side, an F3 primer having a sequence identical with that of the F3 region, a BIP primer having a sequence complementary to the B2c region in the 3' terminal side and a sequence identical with that of the B1c region in the 5' terminal side, and a B3 primer having a sequence complementary to the B3c region,
the FIP primer and BIP primer are selected from the primer sets 1 to 4 shown in Table 3;
the F3 primer binds to a region within 60 bases from the 5' terminal of F2 region of the target nucleic acid; and
the B3 primer binds to a region within 60 bases from the 3' terminal of B2c region of the target nucleic acid.

26. The nucleotide probe according to claim 25, **characterized in that** the wild-type nucleotide probe has a sequence of SEQ ID No. 38, 39 or 40, or a sequence complementary thereto, and the variant nucleotide probe has a sequence of SEQ ID No. 43, 44 or 45, or a sequence complementary thereto.

27. The nucleotide probe according to claim 25, **characterized in that** the probes are immobilized on a support (1, 11).

28. A nucleotide probe for detecting a genotype of a single-nucleotide polymorphism T3010C of the SAA1 gene from an amplification product obtained by amplification of a target nucleotide by using a nucleotide primer set,
**characterized by** comprising:
a nucleotide probe selected from a wild-type nucleotide probe and a variant nucleotide probe,
wherein:
the wild-type nucleotide probe is complementary to the wild-type amplification product and has a Tm value of 55 to 70°C, and the single-nucleotide polymorphism C3010T site is located inside by three or more bases from the terminal thereof, and
the variant nucleotide probe is complementary to the variant amplification product and has a Tm value of 55 to 71°C, and the single-nucleotide polymorphism C3010 site is located inside by three or more bases from the terminal thereof; and wherein
when the target nucleotide has F3 region, F2 region and F1 region in turn from a 5' terminal and B3c region, B2c region and B1c region in turn from a 3'-terminal,
when the nucleotide primer set includes an FIP primer having a sequence identical with that of the F2 region in the 3' terminal side and a sequence complementary to the F1 region in the 5' terminal side, an F3 primer having a sequence identical with that of the F3 region, a BIP primer having a sequence complementary to the B2c region in the 3' terminal side and a sequence identical with that of the B1c region in the 5' terminal side, and a B3 primer having a sequence complementary to the B3c region,
the FIP primer and BIP primer are selected from the primer sets 1 to 4 shown in Table 3;
the F3 primer binds to a region within 60 bases from the 5' terminal of an F2 region of the target nucleic acid; and
the B3 primer binds to a region within 60 bases from the 3' terminal of a B2c region of the target nucleic acid.

29. The nucleotide probe according to claim 28, **characterized in that** the wild-type nucleotide probe has a sequence of SEQ ID No. 48, 49 or 50, or a sequence complementary thereto, and the variant nucleotide probe has a sequence of SEQ ID No. 53, 54, 55 or 56, or a sequence complementary thereto.

30. The nucleotide probe according to claim 28, **characterized in that** the probes are immobilized on a support (1, 11).

31. A method detecting genotypes of single-nucleotide polymorphisms C-13T, C2995T and T3010C of the SAA1 gene simultaneously, **characterized by** comprising the steps of:
obtaining an amplification product by amplifying a target nucleic acid by using the primer set 2 shown in Table 2;
obtaining an amplification product by amplifying the target nucleic acid by using the primer set 3 shown in Table 3;
mixing the amplification products to prepare a liquid mixture;
hybridizing the amplification products to nucleotide probes by bringing the liquid mixture into contact with a nucleotide probe-immobilized support carrying a wild-type nucleotide probe for C-13T having a sequence of SEQ ID No. 28 or a sequence complementary thereto, a variant nucleotide probe for C-13T having a sequence of SEQ ID No. 33 or a sequence complementary thereto, a wild-type nucleotide probe for C2995T having a sequence of SEQ ID No. 38 or a sequence complementary thereto, a variant nucleotide probe for C2995T having a sequence of SEQ ID No. 43 or a sequence complementary thereto, a wild-type nucleotide probe for T3010C having a sequence of SEQ ID No. 50 or a sequence complementary thereto, and a variant nucleotide probe for T3010C having the sequence of SEQ ID No. 55 or a sequence complementary thereto immobilized thereon;
measuring amounts of the amplification product bound to the respective nucleotide probes;
comparing the amounts of the amplification products bound respectively to the wild-type nucleotide probe for C-13T and variant nucleotide probe for C-13T;
comparing the amounts of the amplification products bound respectively to the wild-type nucleotide probe for C2995T and variant nucleotide probe for C2995T; and
comparing the amounts of the amplification products bound respectively to the wild-type nucleotide probe for T3010C and variant nucleotide probe for T3010C.


SEQUENCE LISTING

<110> Kabushiki Kaisha Toshiba

<120> Nucleic acid primer sets and probe for detection of genotype of Serum Amyloid
A1 (SAA1)

<130> EPP99487

<150> JP 2007-117346
<151> 2007-04-26

<160> 59

<170> PatentIn version 3.1

<210> 1
<211> 39
<212> DNA
<213> Artificial

<220>
<223> FIP-1 Primer

<400> 1
cagtggtttc ttcatcccgc aggcacatct tgttccctc 39

<210> 2
<211> 42
<212> DNA
<213> Artificial

<220>
<223> BIP-1 Primer

<400> 2
ggaaggctca gtataaatag catagctgag ctgcgggtcc ct 42

<210> 3
<211> 42
<212> DNA
<213> Artificial

<220>
<223> BIP-2 Primer

<400> 3
ggaaggctca gtataaatag cagtgctgta gctgagctgc gg 42

<210> 4
<211> 43
<212> DNA
<213> Artificial

<220>
<223> BIP-3 Primer

<400> 4
ggaaggctca gtataaatag caacctgatc tgtgctgtag ctg 43


<210> 5
<211> 38
<212> DNA
<213> Artificial

<220>
<223> BIP-4 Primer

<400> 5
ggaaggctca gtataaattg tagctgagct gcgggtcc 38


<210> 6
<211> 38
<212> DNA
<213> Artificial

<220>
<223> BIP-5 Primer

<400> 6
ggaaggctca gtataaatgt gctgtagctg agctgcgg 38


<210> 7
<211> 39
<212> DNA
<213> Artificial

<220>
<223> BIP-6 Primer

<400> 7
ggaaggctca gtataaatac ctgatctgtg ctgtagctg 39

<210> 8
<211> 41
<212> DNA
<213> Artificial

<220>
<223> BIP-7 Primer

<400> 8
ggaaggctca gtataaatag cactcctcac ctgatctgtg c 41

<210> 9
<211> 40
<212> DNA
<213> Artificial

<220>
<223> BIP-8 Primer

<400> 9
ggaaggctca gtataaatag cacttggtgt gctcctcacc 40

<210> 10
<211> 42
<212> DNA
<213> Artificial

<220>
<223> BIP-9 Primer

<400> 10
ggaaggctca gtataaatag caaaaatcac tccttggtgt gc 42

<210> 11
<211> 32
<212> DNA
<213> Artificial

<220>
<223> FIP-2 Primer

<400> 11
ctgtcagggc aggagacctc cgcagcctca gc 32

<210> 12
<211> 33

<212>  DNA
<213>  Artificial

<220>
<223>  FIP-3 Primer

<400>  12
gctgtcaggg caggagaccc cgcagcctca gcc                    33


<210>  13
<211>  44
<212>  DNA
<213>  Artificial

<220>
<223>  FIP-4 Primer

<400>  13
ctgcaggtca tttcccctac atccaggaac ttgtcttaga ccgt                    44


<210>  14
<211>  18
<212>  DNA
<213>  Artificial

<220>
<223>  F3-1 Primer

<400>  14
gtctcctgcc ctgacagc                    18


<210>  15
<211>  19
<212>  DNA
<213>  Artificial

<220>
<223>  F3-2 Primer

<400>  15
tcaggagctg gcttcaaag                    19


<210>  16
<211>  20
<212>  DNA
<213>  Artificial

<220>
<223>  F3-3 Primer

<400>  16
caggcacatc ttgttccctc                                20

<210>  17
<211>  19
<212>  DNA
<213>  Artificial

<220>
<223>  B3-1 Primer

<400>  17
actccttggt gtgctcctc                                19

<210>  18
<211>  24
<212>  DNA
<213>  Artificial

<220>
<223>  B3-2 Primer

<400>  18
aatgataatc ttgttgggaa agag                                24

<210>  19
<211>  32
<212>  DNA
<213>  Artificial

<220>
<223>  FIP-1 Primer

<400>  19
cgtcccagga gctctgctcg ggggaactat ga                                32

<210>  20
<211>  39
<212>  DNA
<213>  Artificial

<220>

<223>  BIP-1 Primer

<400>  20
ctgttcatcc agcctagggg gatgaaaaca ctgggcacc                    39


<210>  21
<211>  33
<212>  DNA
<213>  Artificial

<220>
<223>  FIP-2 Primer

<400>  21
cgtcccagga gctcctgctc gggggaacta tga                    33


<210>  22
<211>  28
<212>  DNA
<213>  Artificial

<220>
<223>  FIP-3 Primer

<400>  22
cgtcccagga gctcagggga cctggggg                    28


<210>  23
<211>  29
<212>  DNA
<213>  Artificial

<220>
<223>  FIP-4 Primer

<400>  23
cgtcccagga gctccagggg acctggggg                    29


<210>  24
<211>  20
<212>  DNA
<213>  Artificial

<220>
<223>  F3 Primer

<400> 24
ccaattacat cggctcagac 20

<210> 25
<211> 17
<212> DNA
<213> Artificial

<220>
<223> B3 Primer

<400> 25
tgagcaatag cccaccc 17

<210> 26
<211> 14
<212> DNA
<213> Artificial

<220>
<223> Negative Controle Probe

<400> 26
gtgctgcagg tgcg 14

<210> 27
<211> 14
<212> DNA
<213> Artificial

<220>
<223> C-13T Wild Type Probe

<400> 27
caccgctccc tggc 14

<210> 28
<211> 16
<212> DNA
<213> Artificial

<220>
<223> C-13T Wild Type Probe

<400> 28
ccaccgctcc ctggca 16

<210> 29
<211> 17
<212> DNA
<213> Artificial

<220>
<223> C-13T Wild Type Probe

<400> 29
gccaccgctc cctggca 17

<210> 30
<211> 18
<212> DNA
<213> Artificial

<220>
<223> C-13T Wild Type Probe

<400> 30
agccaccgct ccctggca 18

<210> 31
<211> 20
<212> DNA
<213> Artificial

<220>
<223> C-13T Wild Type Probe

<400> 31
cagccaccgc tccctggcag 20

<210> 32
<211> 15
<212> DNA
<213> Artificial

<220>
<223> C-13T Mutation Type Probe

<400> 32
caccgttccc tggca 15

<210>  33
<211>  17
<212>  DNA
<213>  Artificial

<220>
<223>  C-13T Mutation Type Probe

<400>  33
ccaccgttcc ctggcag                                17


<210>  34
<211>  18
<212>  DNA
<213>  Artificial

<220>
<223>  C-13T Mutation Type Probe

<400>  34
agccaccgtt ccctggca                               18


<210>  35
<211>  19
<212>  DNA
<213>  Artificial

<220>
<223>  C-13T Mutation Type Probe

<400>  35
cagccaccgt tccctggca                              19


<210>  36
<211>  20
<212>  DNA
<213>  Artificial

<220>
<223>  C-13T Mutation Type Probe

<400>  36
cagccaccgt tccctggcag                             20


<210>  37
<211>  13

<212>   DNA
<213>   Artificial

<220>
<223>   C2995T Wild Type Probe

<400>   37
ggggtgcctg ggc                                13


<210>   38
<211>   14
<212>   DNA
<213>   Artificial

<220>
<223>   C2995T Wild Type Probe

<400>   38
gggggtgcct gggc                               14


<210>   39
<211>   15
<212>   DNA
<213>   Artificial

<220>
<223>   C2995T Wild Type Probe

<400>   39
tgggggtgcc tgggc                              15


<210>   40
<211>   16
<212>   DNA
<213>   Artificial

<220>
<223>   C2995T Wild Type Probe

<400>   40
ctgggggtgc ctgggc                             16


<210>   41
<211>   17
<212>   DNA
<213>   Artificial

<220>
<223>  C2995T Wild Type Probe

<400>  41
cctgggggtg cctgggc                    17


<210>  42
<211>  14
<212>  DNA
<213>  Artificial

<220>
<223>  C2995T Mutation Type Probe

<400>  42
gggggtgtct gggc                    14


<210>  43
<211>  16
<212>  DNA
<213>  Artificial

<220>
<223>  C2995T Mutation Type Probe

<400>  43
ctgggggtgt ctgggc                    16


<210>  44
<211>  17
<212>  DNA
<213>  Artificial

<220>
<223>  C2995T Mutation Type Probe

<400>  44
cctgggggtg tctgggc                    17


<210>  45
<211>  18
<212>  DNA
<213>  Artificial

<220>

<223>   C2995T Mutation Type Probe

<400>   45
acctgggggt gtctgggc                                18



<210>   46
<211>   20
<212>   DNA
<213>   Artificial

<220>
<223>   C2995T Mutation Type Probe

<400>   46
ggacctgggg gtgtctgggc                                20



<210>   47
<211>   19
<212>   DNA
<213>   Artificial

<220>
<223>   T3010C Wild Type Probe

<400>   47
gttacctgat cacttctgc                                19



<210>   48
<211>   21
<212>   DNA
<213>   Artificial

<220>
<223>   T3010C Wild Type Probe

<400>   48
cagttacctg atcacttctg c                                21



<210>   49
<211>   22
<212>   DNA
<213>   Artificial

<220>
<223>   T3010C Wild Type Probe

<400>  49
ccagttacct gatcacttct gc                    22

<210>  50
<211>  23
<212>  DNA
<213>  Artificial

<220>
<223>  T3010C Wild Type Probe

<400>  50
tccagttacc tgatcacttc tgc                    23

<210>  51
<211>  26
<212>  DNA
<213>  Artificial

<220>
<223>  T3010C Wild Type Probe

<400>  51
tccagttacc tgatcacttc tgcrgc                    26

<210>  52
<211>  17
<212>  DNA
<213>  Artificial

<220>
<223>  T3010C Mutation Type Probe

<400>  52
ttacctgatc gcttctg                    17

<210>  53
<211>  19
<212>  DNA
<213>  Artificial

<220>
<223>  T3010C Mutation Type Probe

<400>  53
gttacctgat cgcttctgc                    19

<210> 54
<211> 20
<212> DNA
<213> Artificial

<220>
<223> T3010C Mutation Type Probe

<400> 54
agttacctga tcgcttctgc 20


<210> 55
<211> 21
<212> DNA
<213> Artificial

<220>
<223> T3010C Mutation Type Probe

<400> 55
cagttacctg atcgcttctg c 21


<210> 56
<211> 22
<212> DNA
<213> Artificial

<220>
<223> T3010C Mutation Type Probe

<400> 56
ccagttacct gatcgcttct gc 22


<210> 57
<211> 25
<212> DNA
<213> Artificial

<220>
<223> T3010C Mutation Type Probe

<400> 57
tccagttacc tgatcgcttc tgcrg 25

<210> 58
<211> 24
<212> DNA
<213> Artificial

<220>
<223> LFc Primer

<400> 58
gtcatttatc ccagttgtgc aacc 24

<210> 59
<211> 16
<212> DNA
<213> Artificial

<220>
<223> LBc Primer

<400> 59
agcctggctg aatggg 16